# EUROPEAN PATENT APPLICATION

(11) **EP 2 929 800 A1**
(43) Date of publication of application: **14.10.2015**
(21) Application number: 12889710.5
(22) Date of filing: 06.12.2012
(51) Int. Cl.: A45D 24/00

(54) **COMB**

(71) Applicant: Park Way Co., Ltd., Tokyo 152-0034 (JP)
(72) Inventor: PARK Young-soo, Tokyo 152-0034 (JP)
(74) Representative: Müller Hoffmann & Partner
(86) International application number: PCT/JP2012/081705
(87) International publication number: WO 2014/087528

(57) **Abstract**

A parting line in hair is suitably formed and a length of head hair is measured at a stable posture. In a comb 1, a concaved one end dented portion 3d is formed in one end outer edge surface 3c of one end edge comb part 3. A tip end of the one end edge comb part 3 is pressed against a scalp part where the parting line is to be formed, and a hair bundle divided outside the one end edge comb part 3 is pinched between the one end edge comb part 3 and a finger. Since part of the pinched hair bundle is spread naturally inside the one end dented portion 3d, a stuck feeling is eliminated, and the one end edge comb part 3 is smoothly moved along the parting line without the pinched hair bundle being a resistance, to form the parting line. Further, in the comb 1, a plurality of through-holes 7 are formed in the comb main body 2 at a predetermined interval. In the case of measuring the head hair, the one end outer edge surface 3c is pressed against the scalp, and the measurements of the length of the head hair are carried out based on the through-hole 7, while securing a two-point contact state in which outer edge portions 3e and 3f on both sides of the one end dented portion 3d contact with the scalp.

## Description

### TECHNICAL FIELD

The present invention relates to improve efficiency in forming a parting line of head hair at the time of hairdressing, and which is capable of assisting cutting of the head hair by allowing correct and easy measurements of the length of the head hair.

### BACKGROUND ART

Conventionally, combs are generally used for hairdressing, and at barber shops and beauty salons etc., combs may be used for sorting out head hair to be cut. In barber shops and beauty salons etc., when a dividing line (referred to as a "parting line") is formed during hairdressing, an edge comb part (thicker than comb teeth) having a tapered shape provided at an end of a comb main body may be used, without using a plurality of comb teeth.

Fig. 23(a) illustrates a comb D1 which is one example of a conventional comb. The comb D1 has an edge comb part D1a with a tapered tip, which projects in parallel to a plurality of comb teeth D1b. Fig. 23(b) illustrates a parting process by using the edge comb part D1a. The tip end of the edge comb part D1a is pressed against a part of a scalp Ha where a parting line W is to be formed, and a finger F of a hand which does not hold the comb D1 is pressed against the edge comb part D1a to pinch a hair bundle, which is divided outside the edge comb part D1a, between the edge comb part D1a and the finger F. Then, in such a state, the edge comb part D1a is advanced along a line at which the parting line W is to be formed to sequentially divide a predetermined amount of the head hair into two parts along the parting line W on the scalp Ha, and thereby forming the parting line.

On the other hand, conventionally, there is a comb having a comb main body from which a plurality of comb teeth are extended, and a scale for measurements of length is drawn on the comb main body to allow measurements of the length of the head hair (refer to the following Patent Documents 1 to 3). Moreover, the following Patent Document 4 discloses a comb in which a dot scale is formed on the comb main body or a grip part thereof by burning with a laser beam to allow measurements of the length of the head hair by the scale.

Further, the following Cited Document 5 discloses a comb in which a plurality of through-holes are formed in a side surface of a comb main body at a predetermined interval to allow measurements of the length of the head hair by the through-holes. Further, the following Cited Document 6 discloses a comb in which a plurality of tapered end parts are formed in a side surface of a comb main body at a predetermined interval to allow measurements of the length of the head hair by the taperd end parts.

### [Reference Documents of Conventional Art]

### Patent Documents

Patent Document 1: Japanese Unexamined Utility Model Application Publication No. S57-012104U1
Patent Document 2: Japanese Unexamined Patent Application Publication No. 2007-325889A
Patent Document 3: Japanese Unexamined Utility Model Application Publication No. S53-102798U1
Patent Document 4: Japanese Registered Utility Model Application Publication No. 3107399U
Patent Document 5: Japanese Unexamined Utility Model Application Publication No. H06-066504U1
Patent Document 6: Japanese Unexamined Patent Application Publication No. 2005-304720A

### DISCLOSURE OF THE INVENTION

### [Problems to be Solved by the Invention]

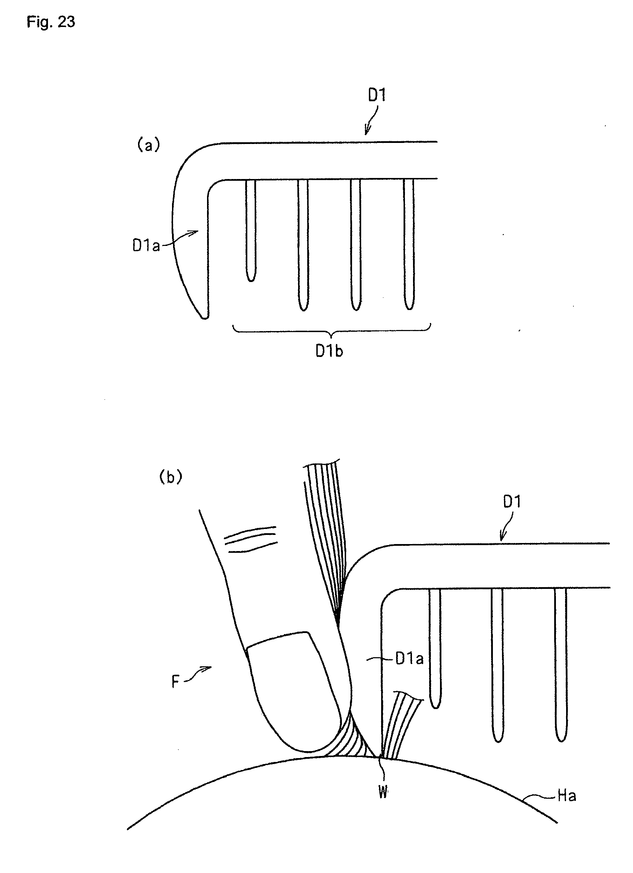

As illustrated in Fig. 23(b), the hair bundle divided outside the edge comb part D1a by the tip end of the edge comb part D1a is pinched between the edge comb part D1a and the finger F. However, since the finger F and the edge comb part D1a are opposed to each other over a wide range along the outer circumference of the edge comb part D1a, the predetermined amount of the hair bundle which is pinched is stuck between the finger F and the edge comb part D1a. Thus, the hair bundle in the state where it is stuck between the finger F and the edge comb part D1a becomes resistance of the edge comb part D1a advancing Therefore, a problem that the resistance disturbs the smooth movement of the edge comb part D1a along the line at which the parting line W is to be formed arises. In addition, since moving the edge comb part D1a smoothly becomes impossible, part of the hair bundle, which is stuck and caught, escapes from the gap between the edge comb part D1a and the finger F and remains near the parting line W. By such a remaining hair part, the boundary of the parting line W becomes obscure, and such a problem that the beautiful appearance of the parting line is spoiled arises.

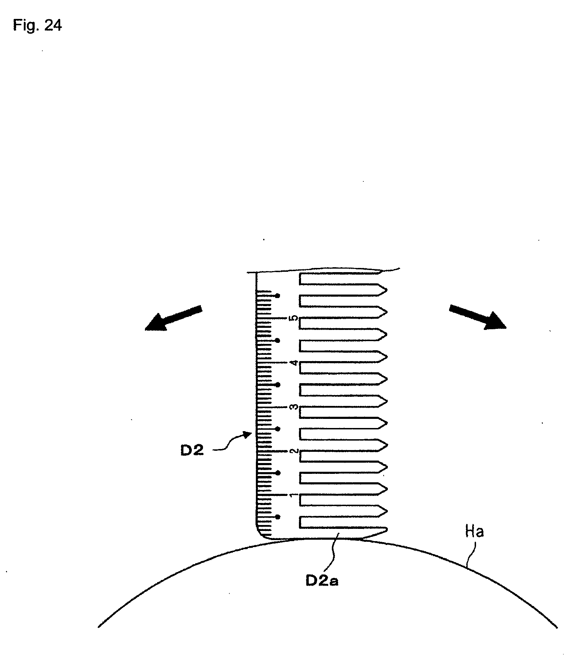

On the other hand, when measuring the length of the head hair, if it is assumed that the comb having the scale as disclosed in Patent Documents 1 to 3 described above is used, it is common that the outer edge surface of the edge comb part of the comb (an outer circumferential part which is a tip end surface of the entire comb) is formed linearly or curved to be an outward convex. Thus, for example, as illustrated in Fig. 24, in order to measure the length of the head hair from the scalp Ha, when the outer edge surface of an edge comb part D2a provided at one end of a comb D2 is pressed against the scalp Ha, the linear outer circumferential part of the edge comb part D2a contacts the curved surface scalp Ha by one-point contacting state. Therefore, since the comb D2 under the measurement becomes in an unstable posture in which the comb D2 shakes largely centering on the contacting location (it shakes in black arrow directions illustrated in Fig. 24), there is a problem that stable measurements cannot be carried out.

Further, since the comb according to Patent Document 4 does not originally have the shape to be pressed against the scalp at the edge comb part, the comb is unsuitable for measuring the length of the head hair from the scalp. Moreover, since both the combs according to Patent Documents 5 and 6 have the shape in which the outer edge surface of the edge comb part is curved to be the outward convex, if the outer edge surface of the edge comb part is pressed against the scalp, the outwardly convex curved surfaces contact to each other. Therefore, such combs are in their more unstable posture than the combs according to Patent Documents 1 to 3, and there is a problem that stable measurements cannot be carried out.

Practical skill assignments in which the head hair pulled out at a predetermined angle from the scalp is cut by a specified length may be assigned, for example, for practical skill instructions at a barber school or a beauty school, or practical skill official examinations of hairdressing and beauty. In such a case, if the angle from which the head hair is pulled out from the scalp changes, the cutting length also changes naturally, so it becomes a very important matter in the practical skill problems to determine the pulled-out angle of the hair with respect to the scalp. However, since the pulled-out angle of the head hair with respect to the scalp cannot be measured by the combs according to Patent Documents 1 to 6 described above, the combs are not effective for the practical skill assignments described above.

Further, the comb is generally held variously depending on users, some users who hold the comb main body from left and right by fingertips, and some users who hold the comb by hooking the comb at the roots of his/her fingers etc. The comb according to Cited Document 6 is configured so that the tapered end part, provided in the comb main body in order to perform the measurements of the length of the head hair, functions as a skid of the fingertip. However, since the tapered end part cannot stop the roots of the fingers etc., there is also a problem that user-friendliness is not enough for some users who hold the comb by hooking the comb at the roots of the fingers etc.

The present invention is made in view of the above situations, and one purpose of the present invention is to provide a comb which reduces a stuck state of a hair bundle between an edge comb part and a user's finger when forming a parting line, by forming a dented portion in an outer edge surface of the edge comb part, to allow a smooth parting work.

Further, another purpose of the present invention is to provide a comb, which allows measurements of a head hair length from a scalp, and which stabilizes the posture of the comb to allow easy and correct measurements of the length of the head hair with the dented portion described above by securing a two-point contacting state at parts remaining on both sides of the dented portion when the outer edge surface of the edge comb part is pressed against the scalp,.

Further, still another purpose of the present invention is to provide a comb which is improved in user-friendliness by using the dented portion described above not only for correct measurements of the length, but also for stopping roots of fingers etc. of the user who holds the comb.

### [Summary of the Invention]

In order to solve the situations described above, a comb in accordance with one aspect of the present invention is provided. The comb includes a comb main body, one end edge comb part formed on one end side in longitudinal directions of the comb main body, and a plurality of comb teeth formed on the comb main body. A dented portion is formed in an outer edge surface between a tip end side and a base end side of the one end edge comb part.

In accordance with the aspect of the present invention, since the dented portion is formed in the outer edge surface of the one end edge comb part (the outer circumferential part corresponding to one of the tip end surfaces of the entire comb), a space due to the dented portion is formed between the one end edge comb part and the finger which is applied to the one end edge comb part when forming the parting line of the head hair by using the one end edge comb part. Therefore, it becomes possible to spread the hair bundle pinched between the one end edge comb part and the finger, within the space formed by the dented portion, easing the pinched state and decreasig the resistance to advance the one end edge comb part smoothly along the line where the parting line is to be formed. As a result, a beautiful parting line can be formed.

In the comb in accordance with the aspect of the present invention, the comb main body has indexes indicative of distances parallel to the longitudinal directions. The indexes may allow a measurement of a length of head hair based on the indexes as the outer edge surface of the one end edge comb part is pressed against a scalp to contact the outer edge portions that remain on both sides of the dented portion with the scalp.

In accordance with the aspect of the present invention, since the comb main body has indexes for measuring the distances, where the one end edge comb part having the dented portion as described above is formed, when the outer edge surface of the one end edge comb part that is the tip end surface on the one end side of the entire comb is pressed against the scalp, the outer circumferential parts that remain on both sides of the dented portion contact the scalp. Therefore, the state of two-point contact with respect to the scalp can be secured. since the posture of the comb at the time of measurement becomes stabilized due to the two-point contacting state, the length of the head hair can be measured correctly.

In the comb in accordance with the aspect of the present invention, a grip part may be formed on the other end side of the comb main body. An outer edge dented portion may be formed in an outer edge surface of the grip part. The measurements of the length of the head hair may be allowed to be carried out based on the indexes by applying the outer edge surface of the grip part to the scalp to contact the outer edge portions that remain on both sides of the outer edge dented portion with the scalp.

In accordance with the aspect of the present invention, the grip part is formed on the other end side of the comb main body, and the dented portion is formed in the outer edge surface of the grip part (the outer circumferential part that is the other end surface of the entire comb). Thus, when the outer circumferential part of the grip part is pressed against the scalp, the state of two-point contact with respect to the scalp can be secured. Therefore, the measurements of the length of the head hair can be carried out in a stable posture even from the grip part side. Further, since the dented portion formed in the grip part also functions as a hooking portion for the root of the finger for instance, the user-friendliness of the comb can also be improved.

In the comb in accordance with the aspect of the present invention, the comb may include the other end edge comb part formed on the other end side in the longitudinal directions of the comb main body. The other end dented portion may be formed in an outer edge surface between a tip end side and a base end side of the other end edge comb part. The measurements of the length of the head hair may be carried out based on the indexes by applying the outer edge surface of the other end edge comb part to the scalp to contact the outer edge portions that remain on both sides of the other end dented portion with the scalp.

In accordance with the aspect of the present invention, since the dented portion (the other end dented portion) are also formed in the outer edge surface of the other end edge comb part formed on the other end side of the comb main body (the outer circumferential part corresponding to the other end surface of the entire comb), the state of two-point contact with respect to the scalp can be secured even if the outer circumferential part of the other end edge comb part is pressed against the scalp. Thus, by using either side of the comb, the stable measurement can be carried out. Therefore, the correct measurement of the head hair can be carried out from any side of the comb according to user's convenience.

In the comb in accordance with the aspect of the present invention, the outer edge surface of the one end edge comb part may be formed obliquely with respect to the longitudinal directions of the comb main body.

In accordance with the aspect of the present invention, since the outer edge surface of the one end edge comb part is formed obliquely, the comb is in an inclined posture with respect to a normal line direction of the scalp having a curved surface when the outer edge surface of the one end edge comb part is pressed against the scalp in order to measure the head hair. Thus, the length of the head hair can be measured at a predetermined oblique angle with respect to the scalp. Therefore, for example, for practical skill instructions at a barber school or a beauty school, or practical skill official examinations of hairdressing and beauty, in order to manage the case where a practical skill assignment to pull out the hair at a predetermined angle and cut by a predetermined length is given, the comb suitable for the practical skill assignment etc. can be provided if the oblique angle of the outer edge surface of the one end edge comb part is designed to be an angle which is frequently given in the practical skill assignment.

In the comb in accordance with the aspect of the present invention, the outer edge surface of the one end edge comb part and the outer edge surface of the other end edge comb part may be formed obliquely with respect to the longitudinal directions of the comb main body, respectively. An angle of the oblique outer edge surface of the one end edge comb part may be different from an angle of the oblique outer edge surface of the other end edge comb part.

In accordance with the aspect of the present invention, since the oblique angles of the respective outer edge surfaces of the one end edge comb part and the other end edge comb part are different from each other, the measurements of the length of the head hair can be carried out correctly and easily at the two kinds of different angles. Therefore, if the angles of the outer edge surfaces of the edge comb parts at both the ends are designed to be two kinds of angles that are frequently given for the practical skill assignment s etc., respectively, the comb becomes very useful for the practical skill assignments etc.

In the comb in accordance with the aspect of the present invention, the comb main body has first indexes indicative of distances from the one end side of the comb main body and second indexes indicative of distances from the other end side of the comb main body.

In accordance with the aspect of the present invention, since the comb main body has the first indexes indicative of the distances from the one end side of the comb main body and the second indexes indicative of the distances from the other end side, the distances can correctly be measured from both sides of the comb main body by properly using the first indexes and the second indexes.

In the comb in accordance with the aspect of the present invention, the comb main body has first indexes corresponding to a first distance unit system and second indexes corresponding to a second distance unit system.

In accordance with the aspect of the present invention, since the first indexes and the second indexes corresponding to the two kinds of distance unit systems are formed, the measurements of the length of the head hair based on the two kinds of distance unit system can be carried out by the single comb. Note that, as one example of the two kinds of distance unit system, it is preferred that, for example, the first indexes are designed to be in a mm (millimeter) unit system which is used in Japan etc. in many cases, and the second indexes are designed to be in an inch (inch) unit system which is used in Europe, the United States, etc. in many cases.

In the comb in accordance with the aspect of the present invention, the first indexes may be different in appearance from the second indexes.

In accordance with the aspect of the present invention, since the first index and the second index are designed to be different in their appearances, the first index and the second index can be distinguished from each other at a glance, and the measurements of the length of the head hair can smoothly be carried out based on the indexes which the user wants to used, even though there are the two kinds of indexes. Note that, as one example in which there are the first indexes and the second indexes different from each other in their appearances, the first indexes are shown by forming a plurality of concaves and convexes in the back side of the comb main body, and designing the interval (pitch) between the concaves or the convexes to be a predetermined dimension, while the second indexes are indicated by forming a plurality of through-holes in the comb main body, and designing the interval (pitch) between the respective through-holes to be a predetermined dimension.

In the comb in accordance with the aspect of the present invention, a comb part having a plurality of comb teeth may be provided to the one end edge comb part, and the outer edge surface may be formed by tip ends of the respective comb teeth of the comb part and tip ends of the respective comb teeth of the comb part form the outer edge surface.

In accordance with the aspect of the present invention, since the comb part is provided so as to form the outer edge surface of the one end edge comb part, it becomes easy to push the hair aside by the comb part and to press the outer edge surface of the one end edge comb part against the scalp. Therefore, even when the hair is thick or long, so the one end edge comb part is interrupted by the hair and is difficult to reach the scalp, the hair can be divided by the comb part, and the one end edge comb part can certainly be pressed (butted) against the scalp.

In the comb in accordance with the aspect of the present invention, a thickness of the one end edge comb part may be larger at the base end side than the tip end side. A concave portion may be formed at a center part in thickness directions of the one end edge comb part, in the outer edge surface on the base end side of the one end edge comb part.

In accordance with the aspect of the present invention, since the concave portion is formed in the center part in the thickness directions on the base end side of the one end edge comb part which is thicker, three outer circumferential parts remain around the dented portion and the concave portion. As a result, when the outer edge surface of the one end edge comb part is pressed against the scalp, the remaining three outer edge portions contact the scalp, and thereby, the state of three-point contact with respect to the scalp can be secured. Further, the stabilized posture of the comb can be secured, and, thereby, the length of the head hair can be measured easier and more correctly.

In the comb in accordance with the aspect of the present invention, a side dented portion may be formed in the one end edge comb part, in at least one of side surfaces between the tip end side and the base end side.

In accordance with the aspect of the present invention, since the side dented portion is formed in at least one of side surfaces of the one end edge comb part, a space where the hair bundle can be spread is secured also in the side. Thus, when the one end edge comb part is moved to carry out the parting work, it becomes more difficult for the hair bundle divided by the tip end of the one end edge comb part to be stuck. Therefore, the workability in the parting work can further be improved, as well as the working efficiency can also be improved. Note that, if the side dented portion is formed so as to communicate with the dented portion of the outer edge surface, it becomes suitable because an integrated dented space can be secured and a sence of resistance by the pinched hair bundle can be further reduced.

### EFFECTS OF THE INVENTION

In accordance with the aspect of the present invention, the predetermined space can be secured in the gap between the user's finger and the one end edge comb part by forming the dented portion in the one end edge comb part, and thereby reducing the stuck of the hair bundly inside the gap. Therefore, the parting work can smoothly be carried out, and also such a situation that part of the stuck hair bundle remains in the parting line is prevented, and, thus, it is useful for the efficient formation of the beautiful parting line.

In accordance with the aspect of the present invention, the indexes for measuring the distances are indicated in the comb main body from which the one end edge comb part having the dented portion is extended. Thus, when the outer circumferential part of the one end edge comb part that is a comb tip end is pressed against the scalp, since the outer edge portions that remain on both sides of the dented portion contact the scalp, the state of two-point contact with respect to the scalp can be secured, the posture of the comb at the time of measurement is stabilized by the two-point contacting state, and, thereby, the length of the head hair can be correctly measured. In accordance with the aspect of the present invention, since the grip part is formed on the other end side of the comb main body and the dented portion is formed in the outer circumference of the grip part, the state of two-point contact with respect to the scalp can be secured even if the outer circumference of the grip part is pressed against the scalp, and the measurements of the length of the head hair can be carried out at the stable posture even from the grip part side. In addition, the dented portion formed in the grip part can be functioned as a hooking portion, such as for the root of the finger, and, thereby the user-friendliness of the comb can also be improved.

In accordance with the aspect of the present invention, the posture of the comb can be stabilized by the other end dented portion formed in the other end edge comb part also when measuring by the other end side, and, thereby, the user-friendliness of the comb for the measurement of the head hair can be improved.

In accordance with the aspect of the present invention, since the outer edge surface of the one end edge comb part is formed obliquely, the precise measurements of the length of the head hair can be carried out at the oblique angle, and the suitable comb can be provided for the performances of the practical skills in the schools of hairdressing and beauty, or the official examinations.

In accordance with the aspect of the present invention, since the angles of the outer edge surfaces of the comb edges at both the ends are made different from each other, the precise measurements of the length of the head hair can be carried out at the two kinds of oblique angles.

In accordance with the aspect of the present invention, since the first indexes indicative of the distances from the one end side and the second indexes indicative of the distances from the other end side are formed in the comb main body, the length of the head hair can be measured precisely and easily by measuring at either one of both the ends of the comb main body, regardless of the comb's full length.

In accordance with the aspect of the present invention, since the first indexes and the second indexes corresponding to the two kinds of distance unit systems indicated, the measurements of the length of the head hair can be carried out based on the two kinds of distance unit systems with the single comb.

In accordance with the aspect of the present invention, since the first indexes and the second indexes are made different in their appearance from each other, the two kinds of indexes can be distinguished at a glance.

In accordance with the aspect of the present invention, since the comb part is provided in the one end edge comb part, the head hair can be pushed aside by the comb part, and the outer edge surface of the one end edge comb part can be pressed (butted) against the scalp, and, thereby, the comb part is useful for the exact measurements of the length of the hair head. In accordance with the aspect of the present invention, since the concave portion is formed in the center part on the thicker base end side of the one end edge comb part, and the three outer circumferential parts are designed to be remained around the dented portion and the concave portion, the state of three-point contact with the scalp can be secured when the outer edge surface of the one end edge comb part is pressed against the scalp. Thus, the more stabilized posture of the comb can be secured, and, thereby, the length of the head hair can be measured easier and more precisely.

In accordance with the aspect of the present invention, since the side dented portion is formed in at least one of the side surfaces of the one end edge comb part, the space by which the sticking of the hair bundle can be released can be secured also in the side surface. Thus, the workability of the parting work can further be improved when the parting work is carried out by the one end edge comb part.

### BRIEF DESCRIPTION OF THE DRAWINGS

Fig. 1 is a schematic perspective view illustrating a comb according to a 1st embodiment of the present invention.
Fig. 2(a) is a front view illustrating details on one end side in longitudinal directions of the comb, and Fig. 2(b) is a side view illustrating one end outer edge surface of one end edge comb part.
Fig. 3 is a schematic view schematically illustrating a parting work (parting line formation) using the one end edge comb part of the comb according to the present invention.
Fig. 4(a) is a schematic view seen from the side of the head, illustrating a state where the one end edge comb part of the comb is pressed against a scalp, and Fig. 4(b) is a schematic view seen from the front of the head, illustrating a state where the one end edge comb part of the comb is pressed against the scalp.
Fig. 5 is a schematic view seen from above the head, illustrating a state where the one end edge comb part of the comb is pressed against the scalp.
Fig. 6 is an enlarged schematic view of the one end edge comb part illustrating a state where the one end edge comb part of the comb is pressed against the scalp.
Fig. 7 illustrates a comb according to a 2nd embodiment which is provided with a grip part, where Fig. 7(a) is a front view, Fig. 7(b) is a schematic view of a substantial part illustrating one example of a holding method, and Fig. 7(c) is a schematic view of a substantial part illustrating another holding method.
Fig. 8 is a front view of a comb according to a 3rd embodiment which is provided with a bar-shaped grip part.
Fig. 9 is a comb according to a 4th embodiment, where Fig. 9(a) is a front view illustrating a substantial part thereof, and Fig. 9(b) is a schematic view illustrating a state of cutting with a pair of hairdressing scissors positioned by the comb.
Fig. 10 is a comb according to a 5th embodiment, where Fig. 10(a) is a plan view illustrating a substantial part thereof, Fig. 10(b) is a schematic view illustrating a comb tooth with a narrow width, and Fig. 10(c) is a schematic view illustrating a comb tooth with a wide width.
Fig. 11 is a schematic perspective view illustrating a comb according to a 6th embodiment.
Fig. 12 is the comb according to the 6th embodiment, where Fig. 12(a) is a plan view illustrating one end side in longitudinal directions, and Fig. 12(b) is a plan view illustrating the other end side in the longitudinal directions.
Fig. 13 is a comb according to a 7th embodiment, where Fig. 13(a) is a front view illustrating a substantial part on one end side, and Fig. 13(b) is a schematic perspective view illustrating a state where the comb is pressed against the scalp while a comb part thereof pushes the head hair aside.
Fig. 14 is a schematic view illustrating a comb according to an 8th embodiment.
Fig. 15 is the comb according to the 8th embodiment, where Fig. 15(a) is a schematic view seen from the side of the head, illustrating a state where one end edge comb part is pressed against the scalp, and Fig. 15(b) is a schematic view seen from the front of the head, illustrating a state where the one end edge comb part is pressed against the scalp.
Fig. 16 is the comb according to the 8th embodiment, where Fig. 16(a) is a schematic view seen from the side of the head, illustrating a state where the other end edge comb part is pressed against the scalp, and Fig. 16(b) is a schematic view seen from above the head, illustrating a state where the other end edge comb part is pressed against the scalp.
Fig. 17 is a comb according to a 9th embodiment, where Fig. 17(a) is a schematic perspective view illustrating a substantial part on one end side in longitudinal directions, Fig. 17(b) is a side view illustrating an outer edge surface of one end edge comb part, and Fig. 17(c) is a plan view on the one end side.
Fig. 18 is a schematic view illustrating a state where the comb according to the 9th embodiment is pressed against the scalp.
Fig. 19 is a comb according to a 10th embodiment, where Fig. 19(a) is a schematic view illustrating a front elevational view, and Fig. 19(b) is a schematic view illustrating a rear elevational view.
Fig. 20 is a comb according to an 11th embodiment, where Fig. 20(a) is a schematic view illustrating a front view, and Fig. 20(b) is a schematic view illustrating a rear elevational view.
Fig. 21 is a comb according to a 12th embodiment, where Fig. 21(a) is a front view illustrating details on one end side in longitudinal directions of the comb, and Fig. 21(b) is a side view illustrating one end outer edge surface of one end edge comb part.
Fig. 22 illustrates a state where a finger is pressed against the one end edge comb part of the comb according to the 12th embodiment in order to carry out a parting work, where Fig. 22(a) is a schematic view seen from above the comb, and Fig. 22(b) is a schematic view seen from the one end outer edge surface.
Fig. 23(a) is a front view illustrating one end side in longitudinal directions of a conventional comb, and Fig. 23(b) is a schematic view schematically illustrating a parting work in which one end edge comb part of the conventional comb is used.
Fig. 24 is a schematic view illustrating a problem in a state where a conventional comb with a scale is pressed against the scalp.

### MODES FOR CARRYING OUT THE INVENTION

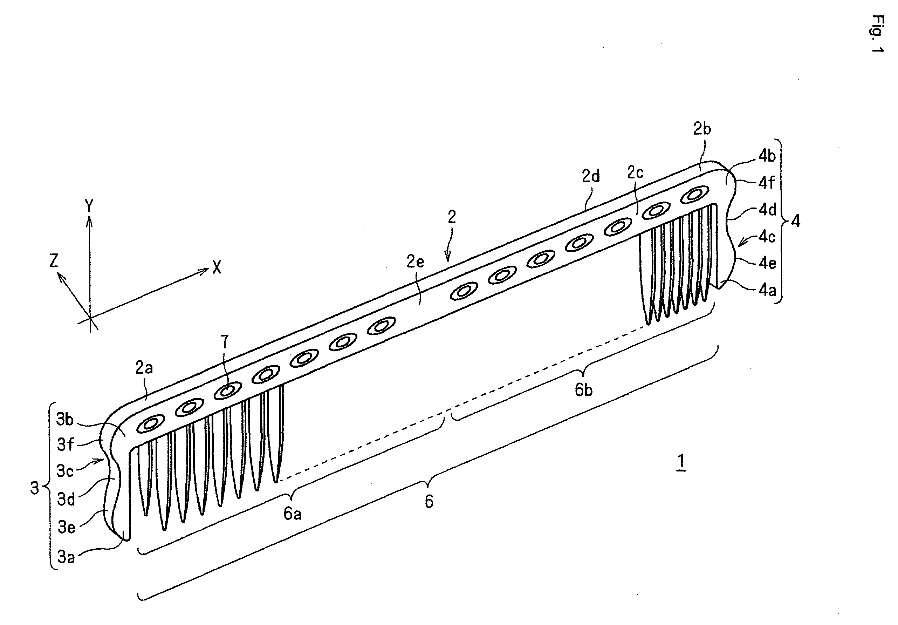

Fig. 1 is a perspective view schematically illustrating the entire part of a comb 1 according to a 1st embodiment of the present invention. The comb 1 of the present invention is able to form used for normal hairdressing etc, and other than that, the comb is capable of forming a parting line of head hair and measure a length of the head hair (referred to as a "hair bundle," and also referred to as a "hair panel") to assist beautiful hairdressing and cutting.

As illustrated in Fig. 1, the comb 1 is made of synthetic resin, and is configured to be provided with one end edge comb part 3 and the other end edge comb part 4 respectively in one end side 2a and the other end part 2b in a square bar shaped comb main body 2, and a plurality of comb teeth 6 are formed so as to extend from the comb main body 2 between the one end edge comb part 3 and the other end edge comb part 4. Note that X-axis directions illustrated in Fig. 1 are directions parallel to longitudinal directions of the comb 1 (the comb main body 2), Y-axis directions which intersect perpendicularly with the X-axis directions are directions parallel to extending directions of the comb teeth 6 and the respective edge comb parts 3 and 4, and Z-axis directions which intersect perpendicularly with the X-axis directions and the Y-axis directions are directions parallel to thickness directions of the comb 1 (the comb main body 2) and the respective edge comb parts 3 and 4 (same for other drawings). Hereinafter, the comb 1 is described in detail.

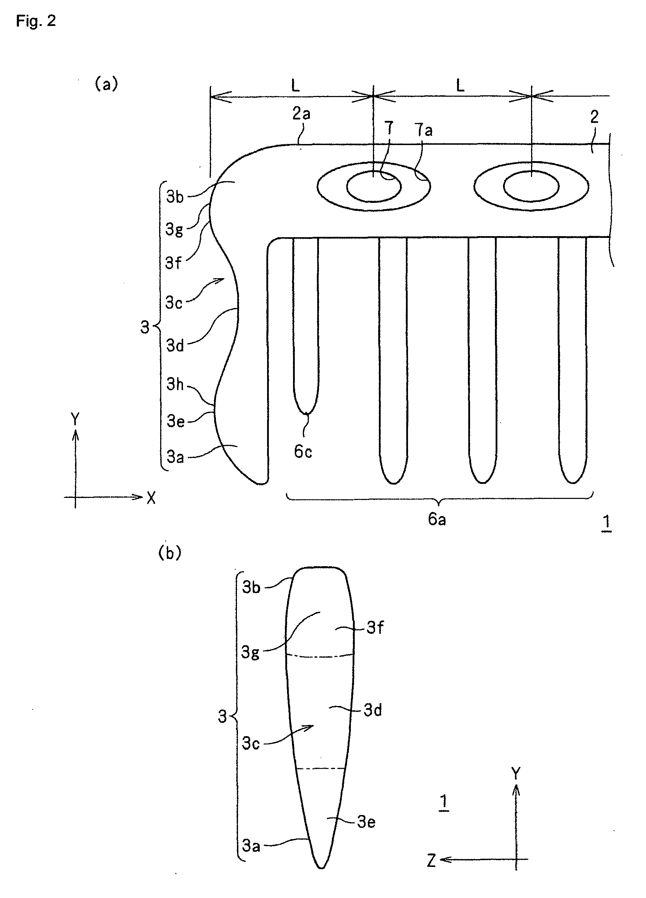

As illustrated in Fig. 1 and Fig. 2(a), the comb main body 2 is formed with a plurality of through-holes 7 which penetrate the comb main body 2 from one side surface 2c to the other side surface 2d, parallel to the Z-axis direction. Each through-hole 7 has an ellipse shape which is elongated in the X-axis direction, and an elliptical counterbore is formed in the circumference of each ellipse. Note that, in the 1st embodiment, the ratio of an inner diameter on the major axis side of the through-hole 7 to an inner diameter on the minor axis side is set to 2 to 1, and, as one example, the inner diameter on the major axis side is set to 5 mm (millimeter) and the inner diameter on the minor axis side is set to 2.5 mm.

Further, as illustrated in Fig. 2(a), the through-hole 7 of the comb main body 2, which is located closest to the one end side 2a, is formed at a location such that the center of the through-hole 7 is separated from an outer circumferential part (a top portion 3g of an outer edge portion) of one end edge comb part 3 by a distance L, and the through-holes 7 which is the second closest to one end side 2a and the subsequent through-holes 7 are formed such that the interval between the centers of the respective through-holes 7 becomes the distance L (note that the center of the through-hole 7 located closest to the other end side 2b is formed at a location from an outer end of the other end edge comb part 4 by the distance L, like the one end side). As for a particular example of the distance L, when using the comb 1 for measurement in a millimeter system of units, the distance L is preferred to be set to 10 mm etc., and when using the comb 1 for measurement in an inch system of units, the distance L is preferred to be set to 1 inch or 0.5 inch etc. The through-holes 7 formed at such an interval correspond to indexes that indicate a predetermined dimension by the distances L (indexes that indicate the distances parallel to the longitudinal directions of the comb main body 2).

The comb main body 2 is formed in the longitudinal direction by the dimension by which the respective through-holes 7 are arrayed at the interval of the distance L as described above. Note that, in order to allow a center portion 2e (refer to Fig. 1) in the longitudinal directions of the comb main body 2 to function as a grip part, no through-hole 7 is formed in the center portion 2e. Further, the comb 1 includes comb teeth 6a of which an interval is wide (as one example of the interval, 3 mm) are provided toward the one end side 2a bordering at the center of the center portion 2e, and comb teeth 6b of which an interval is narrow (as one example of the interval, 1.5 mm) are provided toward the other end side 2b. Note that a comb tooth 6c located closest to the one end edge comb part 3 among the comb teeth 6a on one end side is shortened in its full length comparing to others to facilitate a work in parting line formation of the head hair (parting work) by the one end edge comb part 3, as illustrated in Fig. 2(a).

As illustrated in Fig. 1, and Figs. 2(a) and (b), the one end edge comb part 3 has a tapered shape where its tip end is pointed, extends from the one end side 2a of the comb main body 2 in a direction which intersects perpendicularly with the comb main body 2 (a direction parallel to the Y-axis direction) to serve as a protection by surrounding the comb teeth 6. In the one end edge comb part 3, one end dented portion 3d having a curved shape is formed in an outer circumferential part (a surface that serves as an outer edge) at an intermediate location in the direction parallel to the Y-axis direction from a tapering-shaped tip end 3a side to a base end 3b side which is a coupling side to the comb main body 2. In detail, the one end dented portion 3d which is curved in an arc shape is formed in one end outer edge surface 3c (a surface of the outer edge portion which serves as the outer circumference corresponding to the surface seen in the direction of an arrow indicating the X-axis direction) of the one end edge comb part 3. It is preferred for the curvature of the one end dented portion 3d to be slightly smaller than the smallest curvature part in a head of a common human body, and the radius of curvature is set to 25 mm in the example illustrated in Fig. 2(a).

In the one end edge comb part 3, since the one end dented portion 3d described above is formed in the one end outer edge surface 3c, outer edge portions 3e and 3f remain in a protruded manner, on both sides (the tip end 3a side and the base end 3b side) of the one end dented portion 3d in the Y-axis direction, respectively. Further, the tip end 3a side and the base end 3b side of the one end outer edge surface 3c are formed in a curved shape, respectively, and, thereby, the one end outer edge surface 3c is formed in a continuous curved shape in the Y-axis direction, as illustrated in Fig. 2(a), where the outer edge portion 3e of the tip end 3a is convexed, the one end dented portion 3d at the intermediate location is concaved, and the outer edge portion 3f of the base end 3b is convexed. Note that, as illustrated in Fig. 2(a), the outer edge portion 3e of the tip end 3a is most convexed at a top portion 3h, and the outer edge portion 3f of the base end 3b is most convexed at a top portion 3g. These top portions 3g and 3h are positioned at equivalent locations in the X-axis direction, and these top portions 3g and 3h serve as reference points of the distance L of the through-hole 7 of the comb main body 2, as described above, which is closest to the one end side 2a from the outer end of the one end edge comb part 3. Note that, in Fig. 2(b), in order to distinguish a boundary of the locations where the concaved one end dented portion 3d, and the convexed outer edge portions 3e and 3f are formed, dashed dotted lines are illustrated for convenience, but these dashed dotted lines are imaginary lines and are not actually indicated in the one end comb edge part 3 of the comb 1.

Further, the other end edge comb part 4 provided to the other end side 2b of the comb main body 2 illustrated in Fig. 1 is formed so as to be symmetrical to the one end edge comb part 3 described above with respect to the center portion 2e in the longitudinal direction of the comb main body 2 as the center. In other words, the other end edge comb part 4 includes the other end dented portion 4d which is formed in the other end outer edge surface 4c corresponding to the outer circumference which is located outside in the X-axis direction, at an intermediate location from a tip end 4a to a base end 4b which is located on a coupling side to the comb main body 2, and outer edge portions 4e and 4f remain on both sides (a tip end 4a side and a base end 4b side) of the other end dented portion 4d, respectively.

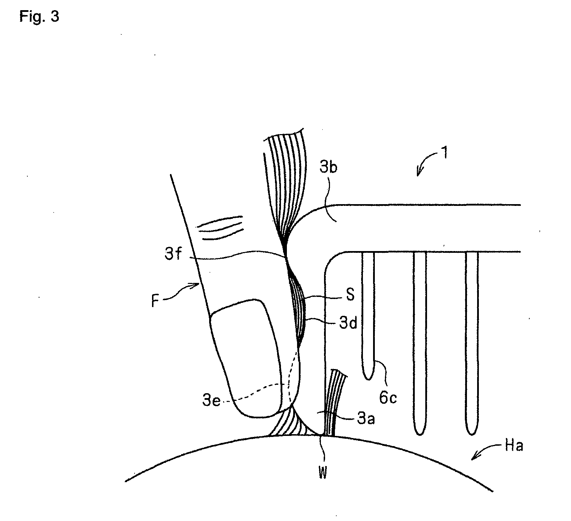

Fig. 3 illustrates a state of forming the parting line by using the one end edge comb part 3 of the comb 1 described above (note that, in order to clarify the feature of the invention, illustration of the through-holes 7 etc. is omitted in Fig. 3). For the formation of the parting line, the tip end 3a of the one end edge comb part 3 is pressed against a location of scalp Ha, where a parting line W is to be formed, to divide the head hair into two parts at the parting line W, and the parting line is formed while pinching the head hair divided to the outside of the one end edge comb part 3 between the outer circumferential part of the one end edge comb part 3 and a finger F. In the comb 1 of this embodiment, since the one end dented portion 3d is formed in the outer circumferential part of the one end outer edge portion 3, a space S between the one end edge comb part 3 and the finger F is produced. Further, since the outer circumference (the one end outer edge surface 3c) of the one end edge comb part 3 is configured so that the outer edge portion 3e on the tip end 3a side and the outer edge portion 3f on the base end 3b side are convexed as described above, the finger F pressed against the one end edge comb part 3 contacts the one end edge comb part 3 centering on the convex parts. Thus, the contacting state between the one end edge comb part 3 and the finger F is reduced in front and rear of the convex parts, compared with the conventional art (refer to Fig. 23(b)).

For this reason, the hair bundle between the one end edge comb part 3 and the finger F is spread naturally within the space S due to the one end dented portion 3d, and the pinching forces in front and rear of the convex parts of the outer edge portion 3e on the tip end 3a side and the outer edge portion 3f on the base end 3b side become weaker compared with the conventional arts. Thus, since the hair bundle pinched between the one end edge comb part 3 and the finger F is released from being stuck over the large range unlike the conventional arts, the resistance feel due to the sticking of the hair bundle is eliminated when the one end edge comb part 3 is moved along the line where the parting line W is to be formed. Therefore, the one end edge comb part 3 can smoothly be moved, and the parting work can efficiently be carried out. Further, by releasing the hair bundle from being stuck, some of the head hair fall off from the hair bundle which is divided outside the one end edge comb part 3, the clear parting line W can be formed, and the beautiful appearance of the parting line can be secured.

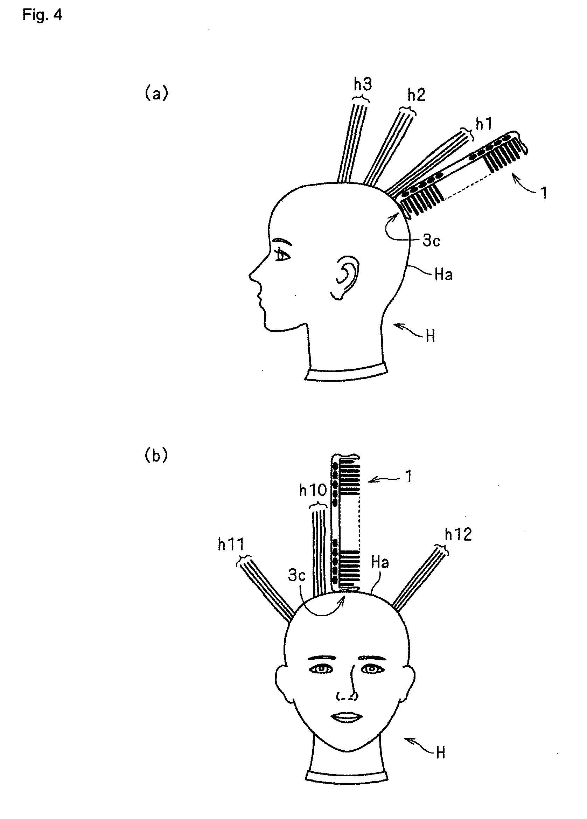

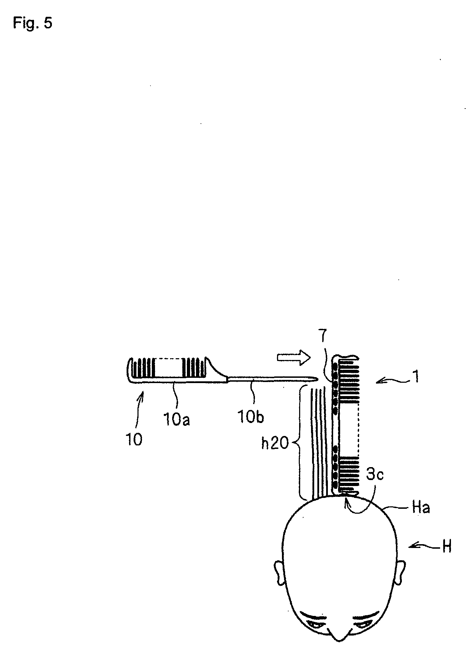

Figs. 4(a) and (b), and Fig. 5 illustrate a situation where the comb 1 described above is used for measurements of the length of the head hair, and illustrates an example where a human head model used for cut practice etc. (normally called a "wig") is used as an object to be cut. However, the state of use for the head of a human body is similar. This human head model H is formed by implanting hair over the entire head part which imitates the human head (note that, in Figs. 4(a) and (b), and Fig. 5, illustration of the entire hair implanted in the head part is omitted and only the necessary hair part is illustrated, in order to clarify the feature of the state of use).

Fig. 4(a) is a view seen from the side of the human head model H, and illustrates a case where measurements are made while one side surface 2c of the comb main body 2 (refer to Fig. 1) is oriented so as to correspond to the side direction of the human head model H. In the measurements, hair bundles (hair panels) h1, h2, and h3 serve as the measuring objects are gathered from the head part respectively by a width of about 10 to 50 mm, and the lengths of these hair bundles h1, h2, and h3 are measured from a scalp Ha using the comb 1. For example, as illustrated in Fig. 4(a), when measuring the hair bundle h1 located at a rear portion of the head part, the hair bundle h1 gathered from the head part is raised from the scalp Ha, and the comb main body 2 of the comb 1 is then placed along the hair bundle h1. At this time, the one end outer edge surface 3c of the one end edge comb part 3 of the comb 1 is pressed (butted) against the scalp Ha which serves as a root part of the hair bundle h1 of the human head model H.

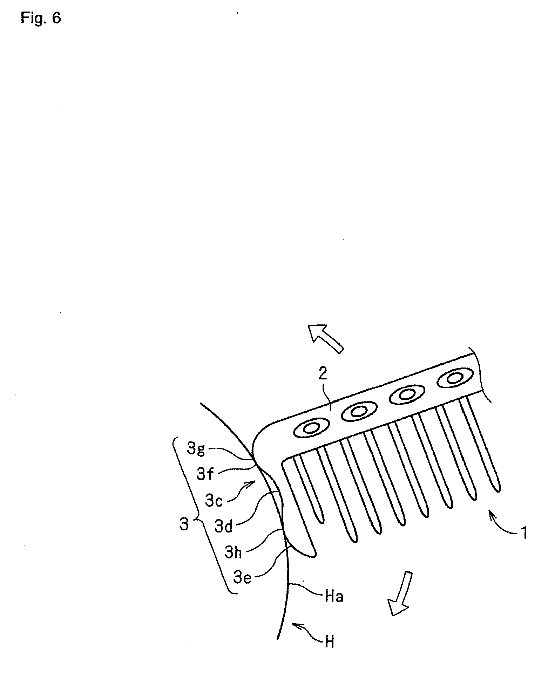

Fig. 6 illustrates the details of the state where the one end outer edge surface 3c of the one end edge comb part 3 of the comb 1 is pressed against the scalp Ha near the rear portion of the head part. Since the one end dented portion 3d described above is formed in the one end outer edge surface 3c of the one end edge comb part 3, when the one end outer edge surface 3c is pressed against the scalp Ha, the outer edge portions 3e and 3f which are convexed on both sides of the one end dented portion 3d contact the scalp Ha (more specifically, they contact mostly at top portions 3h and 3g of the outer edge portions 3e and 3f, respectively). Since the one end dented portion 3d is formed so as to have a curvature slightly smaller than the smallest curvature part in the head part of a common human body, a gap is produced between the one end dented portion 3d and the scalp Ha, and thereby the outer edge portions 3e and 3f on both sides contact the scalp Ha. Therefore, when the comb 1 is pressed against the scalp Ha at the one end outer edge surface 3c of the one end edge comb part 3, the comb 1 becomes in a state of two-point contact at the outer edge portions 3e and 3f to the scalp Ha, and a stable posture of the comb 1 can be maintained without the comb 1 swaying to white arrow directions (left and right directions) illustrated in Fig. 6 (it does not shake in the white arrow directions). Thereby, the comb 1 can allow the measurement of the length of the head hair precisely and easily compared with the conventional comb D2 illustrated in Fig. 24 (the measurements of length are performed by using the plurality of through-holes 7 described above as the indexes each distance L).

Note that other hair bundles h2 and h3 etc. illustrated in Fig. 4(a) can also be measured similarly the hair bundle h1 described above. Of course, the measurement of hair can also be carried out in the unit of, such as one hair or two hairs, other than carrying out by the hair bundle (hair panel) as described above.

Fig. 4(b) is a view seen from the front of the human head model H, and illustrates a case where measurements are made while the one side surface 2c of the comb main body 2 (refer to Fig. 1) is oriented so as to correspond to the front direction of the human head model H. Also in the measurements, of such a case hair bundles h10, h11, and h12 etc. which serve as the measuring objects are gathered from the head part, and the comb 1 which is pressed against the scalp Ha at the one end outer edge surface 3c is oriented along the hair bundles h10, h11, and h12 etc. Thus, the comb 1 can allow measurements similarly the case of Fig. 4(a) described above, and as illustrated in Fig. 6, a state of two-point contact is secured on both sides of the one end dented portion 3d to allow the comb 1 to carry out stable measurements.

Fig. 5 is a view seen from above the human head model H, and illustrates a case where measurements are made while the one side surface 2c of the comb main body 2 (refer to Fig. 1) is oriented so as to correspond to the upward direction of the human head model H. Also in the measurements, of such a case hair bundles h20 etc., which serves as the measuring object, near the rear portion of the head part is gathered from the head part, and the comb 1 is oriented along the hair bundle h20 etc. Thus, similar measurements as the cases of Figs. 4(a) and (b) described above can be carried out. Also in such a case, since the one end outer edge surface 3c of the comb 1 which is pressed against the scalp Ha becomes in the state of two-point contact as illustrated in Fig. 6, it can carry out stable measurements of length. Note that Figs. 4(a) and (b), and Fig. 5 illustrate examples of the measurements of length, and similar measurements are also possible at other parts of the head part.

Further, if a tip end position of the hair which is to be gathered from the head part has been correctly confirmed; it is preferred to use a type of comb 10 called a tail comb, in combination with the comb 1, as illustrated in Fig. 5. The comb 10 of the tail comb type has a bar-shaped grip part 10b projecting from a comb main body 10a, and the entire combination of the comb 1 and the comb 10 becomes an L-shape by inserting the grip part 10b into the through-hole 7 of the comb 1. Thus, it becomes easier to measure the length of the hair compared with the case where the length of the hair is measured only by the through-hole 7 of the comb 1, basing on positional relationship between the tip end of the hair which is the measuring object and the bar-shaped grip part 10b inserted into the through-hole 7. Note that the through-hole 7 of the comb 1 into which the grip part 10b of the comb 10 is inserted is changed suitably according to the length of the hair which is the measuring object. Therefore, if one through-hole 7 is not suitable after once measured, another measurement is made after changing to another through-hole 7 at a suitable position. The method of measurement which combined such a comb 10 with the comb 1 is, of course, also applicable to Figs. 4(a) and (b), and when using the through-holes 7 as the indexes indicative of dimensions, the method has more superiority as compared to the scales of the conventional combs (refer to Patent Documents 1 to 3) in that the method of measurement which combined such a comb 10 with the comb 1 can be realized.

Note that the measurements of the length of the hair of the head (hair) described above are described by the case where the one end outer edge surface 3c of the one end edge comb part 3 is pressed against the scalp Ha. However, even when the other end outer edge surface 4c of the other end edge comb part 3 is pressed (placed) against the scalp Ha, the correct measurements of length can be carried out while the comb 1 is in the stable posture due to the existence of the other end dented portion 4d, similarly the case where the one end outer edge surface 3c is pressed against the scalp Ha. For this reason, the user of the comb 1 can carry out the measurements of length by either one end side 2a or the other end side 2b of the comb main body 2 without caring about the directivity of the comb 1, so the comb 1 has an advantage also in this point compared to the conventional combs (refer to Patent Documents 1 to 3).

Note that the comb 1 according to the 1st embodiment is not limited to the form as described above, and various modifications can be considered. For example, when making the configuration simple, the other end outer edge surface 4c of the other end edge comb part 4 may not be formed with the other end dented portion 4d, but the other end outer edge surface 4c may be formed in a shape similar to the conventional art, and the comb 1 may be configured to have specifications in which only the one end edge comb part 3 is pressed against the scalp in the case of measurement. Further, it is possible to use various forms other than the through-holes 7 as the indexes indicative of the distances in the longitudinal direction of the comb main body 2, and, for example, it is possible to use the scales illustrated in Patent Documents 1 to 3 described above etc.

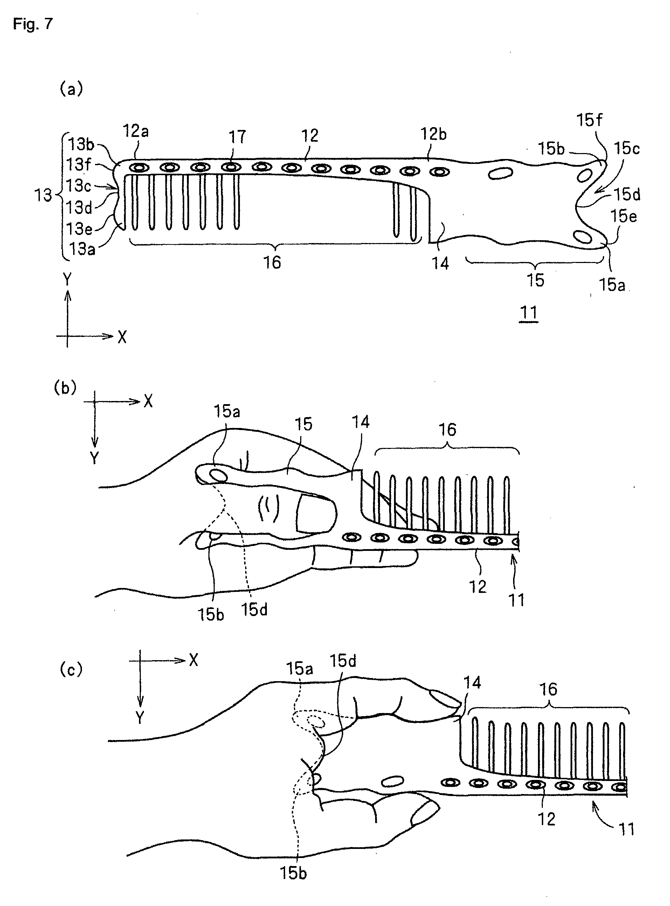

Figs. 7(a), (b), and (c) illustrate a comb 11 according to a 2nd embodiment of the present invention. The comb 11 of the 2nd embodiment is characterized in that a plate-shaped grip part 15 is provided so as to extend in the X-axis direction from the other end edge comb part 14 on the other end side 12b of a comb main body 12, and a dented portion 15d (outer edge dented portion) is formed in an outer edge surface 15c which serves as an outer end of the grip part 15 in the X-axis direction. This dented portion 15d of the outer edge surface 15c is different in the shape from the other end dented portion 4d which is formed in the other end edge comb part 4 of the comb 1 described above, and is deeply dented in a V-shape, instead of the arc shape. Particularly, the depth (the depth in the X-axis direction) of the dented portion 15d has a ratio of about 1:2 to the opening width of the dented portion 15d in the Y-axis direction, and, as one example, the depth is 15 mm and the opening width is 30 mm. Note that, in the outer edge surface 15c of the grip part 15, outer edge portions 15e and 15f exist on one end 15a side and the other end 15b side which are both sides of the dented portion 15d so as to project.

Further, the comb 11 is configured to equivalent to the comb 1 of the 1 st embodiment for other than the parts described above. One end edge comb part 13 is provided on one end side 12a of the comb main body 12 where a plurality of through-holes 17 are formed and from which a plurality of comb teeth 16 are extended. In the one end edge comb part 13, one end dented portion 13d is formed in one end outer edge surface 13c in the direction parallel to the Y-axis direction, at an intermediate location from a tip end 13a to a base end 13b, and outer edge portions 13e and 13f are formed on both sides of the one end dented portion 13d.

First, such a comb 11 can carry out a parting work as illustrated in Fig. 3 by the one end edge comb part 13. In addition, by pressing either the one end outer edge portion 13c of the one end edge comb part 13 or the outer end portion 15c of the grip part 15 against the scalp, stable measurements of length can be carried out at both the ends of the comb 11 in the longitudinal directions due to the existence of the one end dented portion 13d or the dented portion 15d, in a manner equivalent to the comb 1 of the 1 st embodiment described above. In addition, in the comb 11 of the 2nd embodiment, the dented portion 15d of the grip part 15 can also be used as a locking portion for a finger etc. at the time of the user holding the comb 11.

Fig. 7(b) illustrates one example in which the dented portion 15d is used as the hooking portion, and illustrates a suitable example for a type of a user who pinches the comb 11 in the thickness directions (Z-axis directions) as his/her way to hold the comb 11. Particularly, the way to hold the comb 11 is such that the grip part 15 is pinched from both sides thereof in a state in which the comb 11 is hooked by pressing the root of the finger between the thumb and the index finger against the dented portion 15d. In such a case, since the projected outer edge portions 15a and 15b of the grip part 15d is locked on the circumference of the root of the thumb, catching of the thumb becomes better, and such a trouble that the comb 11 falling from the user's hand hardly occurs.

Further, Fig. 7(c) illustrates a suitable example for the type of user who holds the comb 11 by pinching the comb 11 in the width directions (the height direction or the Y-axis direction) as his/her way to hold the comb 11, and the way to hold the comb 11 is to pinch both the ends around the grip part 15 between the thumb and the index finger in a state in which part near the root of the index finger is pressed against the dented portion 15d. In such a case, since the projected outer edge portions 15a and 15b of the grip part 15d is locked so that the outer edge portions 15a and 15b are bitten into the edge part of the palm, such a trouble that the comb 11 falling from the user's hand hardly occurs. Note that the comb 11 according to the 2nd embodiment is not limited to the form as described above, but, for example, the scales illustrated in Patent Documents 1 to 3 described above can also be used as the indexes indicative of the distances in the longitudinal direction of the comb main body 12, instead of the through-holes 17.

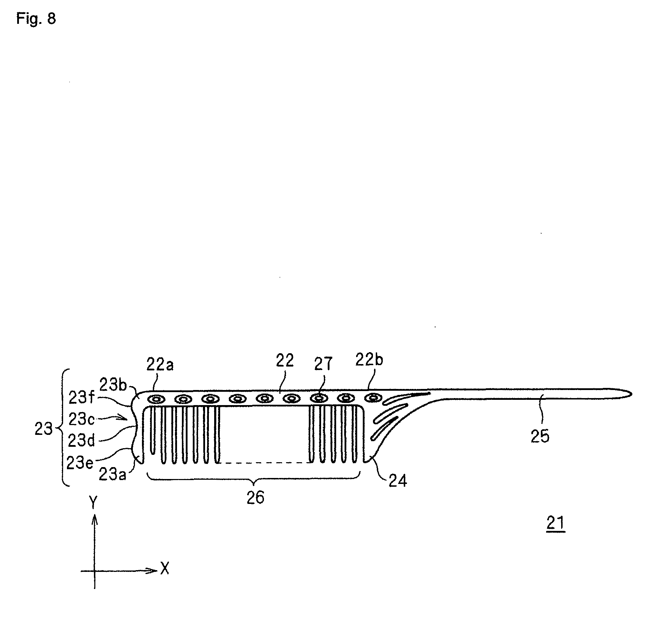

Fig. 8 illustrates a comb 21 according to a 3rd embodiment of the present invention. The comb 21 of the 3rd embodiment is generally a type called a tail comb, and it is characterized in that a bar-shaped grip part 25 extends from the other end edge comb part 24 of a substantially triangular shape provided to the other end side 22b of a comb main body 22.

The comb 21 is formed in a form equivalent to the comb 1 of the 1st embodiment for other than the parts described above. One end edge comb part 23 is formed at one end side 22a of the comb main body 22 where a plurality of through-holes 27 are formed and from which a plurality of comb teeth 26 are extended. In this one end edge comb part 23, one end dented portion 23d is formed in one end outer edge surface 23c in the direction parallel to the Y-axis direction, at an intermediate location from a tip end 23a to a base end 23b, and outer edge portions 23e and 23f are formed on both sides of the one end dented portion 23d.

With such a comb 21, the parting work as illustrated in Fig. 3 and the correct measurements of length at the stable posture as illustrated in Fig. 6 can be carried out similar to the comb 1 of the 1 st embodiment described above, by the one end dented portion 23d of the one end edge comb part 23, while securing the user-friendliness similar to the conventional tail comb by the bar-shaped grip part 25 on the other end part 22b side. Note that it is possible to use, also in the comb 21 of the 3rd embodiment, the scales illustrated in Patent Documents 1 to 3 etc. described above, as the indexes indicative of the distances, instead of the through-holes 27.

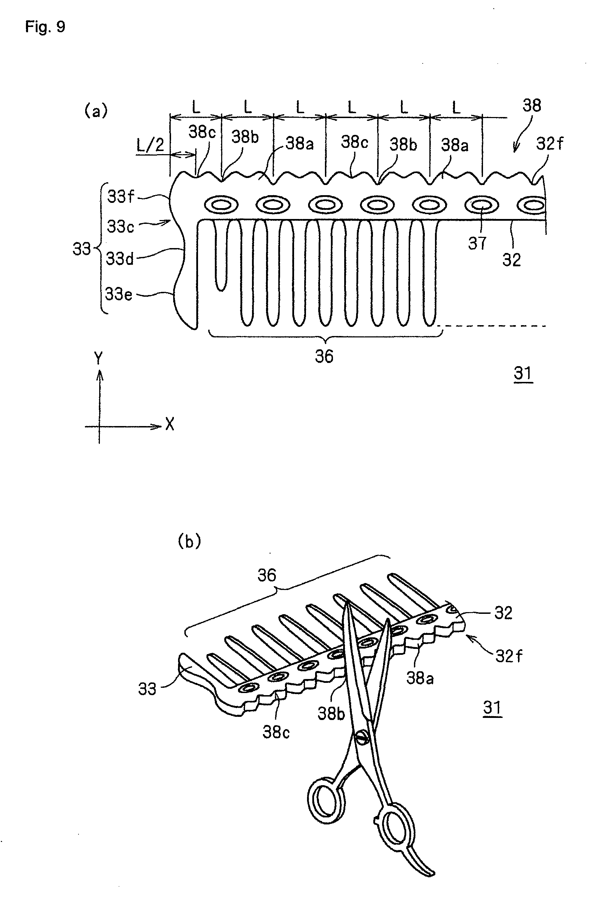

Figs. 9(a) and (b) illustrate a comb 31 according to a 4th embodiment of the present invention, and the comb 31 of the 4th embodiment is characterized in the indexes indicative of dimensions. In other words, the comb 31 is formed with a plurality of through-holes 37 (first indexes) in a comb main body 32 as the indexes indicative of dimensions, similar to the comb 1 of the 1 st embodiment described above. In addition, a plurality of valley portions 38b and auxiliary valley portions 38c are formed in an upper surface 32f of the comb main body 32 so that these valley portions 38b and the auxiliary valley portions 38c are used as indexes (second indexes).

The plurality of valley portions 38b are formed at locations corresponding to the centers of the plurality of through-holes 37, and, thereby, the interval of the valley portions 38b is the distance L (also, the distance from the valley portion 38b closest to the one end edge comb part 33 to the outer edge of the one end edge comb part 33 is also the distance L). The valley portion 38b is dented in a V-shape, the ratio of the opening width and the depth is about 1:1, and the opening width and the depth are about 3 mm, as one example.

Each of the auxiliary valley portions 38c is formed so as to be located in the middle of the adjacent valley portions 38b, and the intervals of the auxiliary valley portions 38c and the valley portions 38b are a distance L/2, respectively (also, the distance from the auxiliary valley portion 38c closest to the one end edge comb part 33 to the outer edge of the one end edge comb part 33 is the distance L/2). Although the auxiliary valley portions 38c are also V-shaped dents, their depth is shallower than the depth of the valley portions 3b described above. As one example, the auxiliary valley portions 38c have an opening width of 3 mm, and a depth of about 1.5 mm. Note that a convex mountain portion 38a is formed between the valley portion 38b and the auxiliary valley portion 38c which are adjacent to each other. A concave-convex part 38 comprised of the valley portions 38b, the auxiliary valley portions 38c, and the mountain portions 38a functions as a locking portion of a fingertip for the type of user who holds the comb while applying his/her fingertip along the upper surface 32f of the comb main body 32.

The comb 31 is equivalent to the comb 1 of the 1 st embodiment for other than the parts described above. For example, the one end edge comb part 33 is formed on one end side of the comb main body 32 from which a plurality of comb teeth 36 are extended. In the one end edge comb part 33, outer edge portions 33e and 33f are formed in one end outer edge surface 33c, on both sides of one end dented portion 33d.

With such a comb 31, the parting work for forming the beautiful parting and the correct measurements of length in the stable posture can be carried out by the one end dented portion 33d of the one end edge comb part 33, similar to the comb 1 of the 1 st embodiment described above. In addition, since, in the comb 31, the valley portions 38b and the auxiliary valley portions 38c are formed in the upper surface 32f of the comb main body 32, the measurements can be made at the upper surface 32f of the comb main body 32 which can easily be placed along the head hair when measuring and, thus, the measurements can be carried out more easily. Further, since the distance L/2 which is a half of the distance L can be measured by the auxiliary valley portions 38c, the measurements can be carried out in more detail by the two kinds of indexes that are the valley portions 38b and the auxiliary valley portions 38c.

In addition, as illustrated in Fig. 9(b), the comb 31 is possible to lock (stop) the back edge portion of one blade of a pair of hairdressing scissors at the valley portion 38b and the auxiliary valley portion 38c. If the back edge portion of the blade of the hairdressing scissors is locked, the hairdressing scissors can be positioned by the valley portion 38b or the auxiliary valley portion 38c in the case of cutting the hair. Therefore, the length to be cut is measured by the valley portion 38b or the auxiliary valley portion 38c, and in the state where the measurement is made, if the back edge portion of the blade of the hairdressing scissors is locked by the valley portion 38b or the auxiliary valley portion 38c to position the hairdressing scissors and the cut is then carried out, the cut can smoothly be carried out at the correct length.

Note that the comb 31 according to the 4th embodiment is not limited to the form as described above, but, for example, if the measurement of the distance L/2 is unnecessary, it is possible to omit the formation of the auxiliary valley portions 38c, and it is also possible to omit the formation of the through-holes 37. In addition, in the comb 31, the other end side of the comb main body 32 is also possible to be formed similar to that of the comb 1 of the 1 st embodiment described above, or it is also possible to provide the grip part similar to the comb 11 of the 2nd embodiment described above, or it is also possible to provide the bar-shaped grip part similar to the comb 21 of the 3rd embodiment described above.

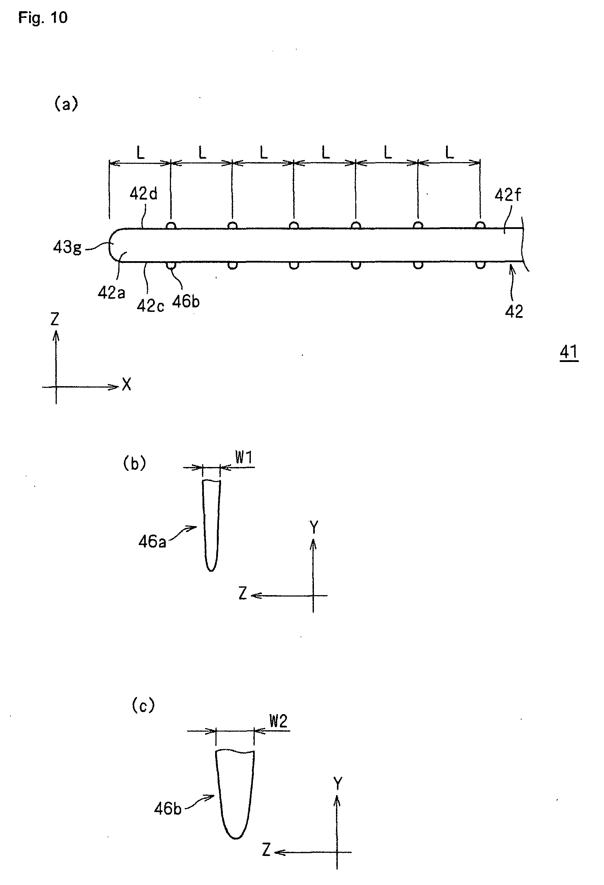

Fig. 10(a) illustrates a comb 41 according to a 5th embodiment of the present invention. The comb 41 of the 4th embodiment has a feature in the indexes indicative of dimensions, and the indexes are configured to be distinguishable from other common comb teeth 46a, by forming the comb teeth located at positions indicative of the distances L, so as to be wider to use them as index comb teeth 46b. Note that the comb 41 has a configuration similar to that of the 1st embodiment, for one end edge comb part and the other end edge comb part.

In other words, as illustrated in Fig. 10(b), the common comb teeth 46a have a dimension W1 at the location where the width is the widest. On the other hand, as illustrated in Fig. 10(c), the index comb teeth 46b have a dimension W2 at the location where the width is the widest (W1<W2). The dimension W2 is set larger than a thickness (a dimension in the Z-axis direction) of the comb main body 42. Thus, as illustrated in Fig. 10(a), in a state where the comb main body 42 is seen from an upper surface 42f thereof, the index comb teeth 46b is configured to be protruded from both side surfaces 42c and 42d of the comb main body 42. Therefore, the location at every distance L can be checked by using the index comb teeth 46b. Note that the index comb tooth 46b closest to one end part 42a side of the comb main body 42 is located at the distance L from a top portion 43g of the outer edge.

Thus, in the comb 41 of the 5th embodiment, since the measurement can be carried out by the unit of the distance L by the index comb teeth 46b which project from the side surfaces 42c and 42d of the comb main body 42, the formation of the through-holes in the side surfaces of the comb main body 42 can be omitted, and the formation of the valley portions etc. in the upper surface of the comb main body 42 as illustrated in Fig. 9(a) can also be omitted. Further, since the interval of the length of the distance L can be confirmed by using the projected index comb teeth 46b not only by viewing but also by the sense of touch such as at a fingertip, it is advantageous that the outline of the length can be grasped only by the sense of touch, without visually checking each time. Note that, also in the comb 41 of the 5th embodiment, the various modifications described in the above 4th embodiment are applicable to the other end side of the comb main body 42.

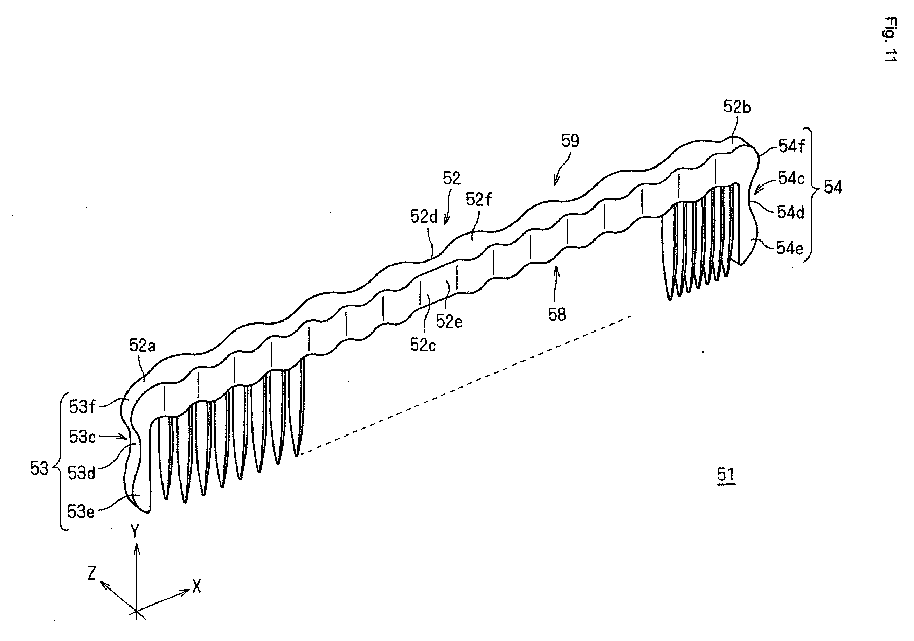

Fig. 11 illustrates a comb 51 according to a 6th embodiment of the present invention. The comb 51 of the 6th embodiment also has a feature in indexes indicative of dimensions. The comb 51 uses first indexes formed in one side surface 52c of a comb main body 52, and second indexes formed in the other side surface 52d as indexes of a different unit system to be applicable to measurements based on two kinds of unit systems by this single comb 51.

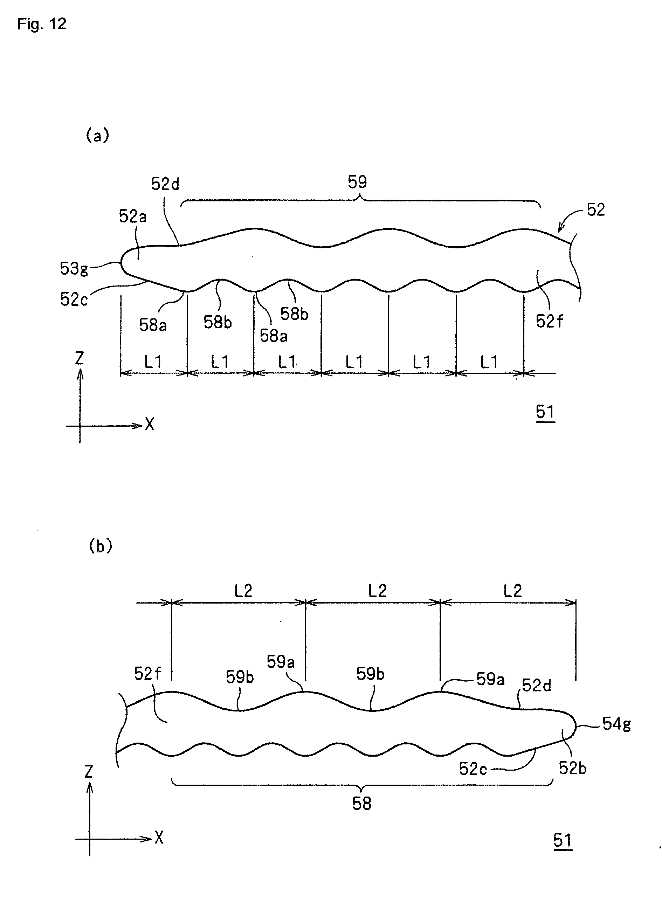

As illustrated in Fig. 11, and Figs. 12(a) and (b), in the comb 51, a curved part 58 which is comprised of a plurality of convex portions 58a and concave portions 58b is formed in the one side surface 52c of the comb main body 52. The interval of the respective convex portions 58a is a distance L1 (refer to Fig. 12(a)), and, the convex portion 58a closest to a top portion 53g of one end outer edge surface 53c of one end edge comb part 53 provided on one end side 52a of the comb main body 52 is located at the distance L1 from a top portion 53g. The distance L1 corresponds to a dimension of the millimeter unit system as a first distance unit system, and is set to 10 mm as one example. Note that the convex portions 58a of the curved part 58 correspond to the first indexes (indexes indicative of the distance from the one end outer edge surface 53c of the comb 51) (further, the interval between the convex portion 58a and the concave portion 58b which are adjacent to each other is a half of L1).

In addition, in the comb 51, a curved part 59 which is comprised of a plurality of convex portions 59a and a plurality of concave portions 59b is formed on the other side surface 52d of the comb main body 52. The interval of the respective convex portions 59a is a distance L2 (refer to Fig. 12(b)), the convex portion 59a closest to a top portion 54g of the other end outer edge surface 54c of the other end edge comb part 54 provided to the other end side 52b of the comb main body 52 is located at the distance L2 from the top portion 54g. This distance L2 corresponds to a dimension of the inch unit system as a second distance unit system, and is set to 1 inch as one example. Note that the convex portions 59a of the curved part 59 correspond to second indexes (indexes indicative of the distance from the other end outer edge surface 54c of the comb 51) (further, the interval of the convex portion 59a and the concave portion 59b which are adjacent to each other is a half of L2).

Note that the dimensions in longitudinal directions of the comb main body 52 of the comb 51 are not formed in the unit dimension exactly corresponding to a 10-mm unit and a 1-inch unit (it is formed in the dimension which produces a fraction in millimeters or inches). Therefore, in the one side surface 52c of the comb main body 52, the convex portion 58a closest to the top portion of the other end outer edge surface 54c of the other end edge comb part 54 is formed so that the dimension from the top portion 54g of the other end outer edge surface 54c is not the distance L1 because of the full length dimension of the comb main body 52. Similarly, in the other side surface 52d of the comb main body 52, the convex portion 59a closest to the top portion of the one end outer edge surface 53c of the one end edge comb part 53 is formed so that the dimension from the top portion 53g of the one end outer edge surface 53c is not the distance L2 because of the full length dimension of the comb main body 52.

In addition, as illustrated in Fig. 11, the comb 51 is provided with one end edge comb part 53 on one end side 52a of the comb main body 52, and this one end edge comb part 53 is formed with outer edge portions 53e and 53f on both sides of one end dented portion 53d formed in the one end outer edge surface 53c, similar to the comb 1 of the 1st embodiment described above. Further, the other end edge comb part 54 provided on the other end side 52b of the comb main body 52 is provided with outer edge portions 54e and 54f on both sides of the other end dented portion 54d formed in the other end outer edge surface 54c. Note that the comb main body 52 is formed with a flat grip part in a center part 52e in longitudinal directions thereof, and an upper surface 52f thereof is also formed so as to be flat. However, valley portions (auxiliary valley portions) and mountain portions may be formed similarly the comb 31 according to the 4th embodiment of Figs. 9(a) and (b), and the interval of the valley portions may be set so as to correspond to either the distance L1 or the distance L2 described above. Alternatively or additionally, they may be indexes indicative of a third distance interval (distance L3) as third indexes.

Such a comb 51 is characterized in that the measurements of length are made based on the two kinds of unit systems. In other words, when the one end outer edge surface 53c of the one end edge comb part 53 is pressed against the scalp, the measurements of length can be carried out based on the unit of the distance L1 or the half of L1 (the unit in mm) by using the convex portions 58a or the concave portions 58b of the curved part 58 formed in the one side surface 52c of the comb main body 52. Further, when the other end outer edge portion 54c of the other end edge comb part 54 is pressed against the scalp, the measurements of length can be carried out based on the unit of the distance L2 or the half of L2 (the unit in inches) by using the convex portions 59a or the concave portions 59b of the curved part 59 formed in the other side surface 52d of the comb main body 52. Note that, also in such measurements, the posture of the comb 51 can be stabilized at the time of the measurements and the length can be correctly measured by the one end dented portion 53d and the other end dented portion 54d. Further, by the comb 51 according to the 6th embodiment, the parting work as illustrated in Fig. 3 can also be suitably carried out by each of the dented portions 53d and 54d.

Note that the comb 51 according to the 6th embodiment is not limited to the form as described above, and as for the two kinds of indexes, it is also possible to suitably use the through-holes illustrated in Fig. 1 etc. described above, the valley portions formed in the comb main body upper surface illustrated in Fig. 9(a), the wider comb illustrated in Fig. 10, or the conventional scales etc., instead of at least one of the convex portion 58a and the convex portion 59a. Note that the indexes of the convex portions 58a and the convex portions 59a formed in the comb main body side surfaces can also be applied to the comb 1 etc. of the 1 st to 5th embodiments described above.

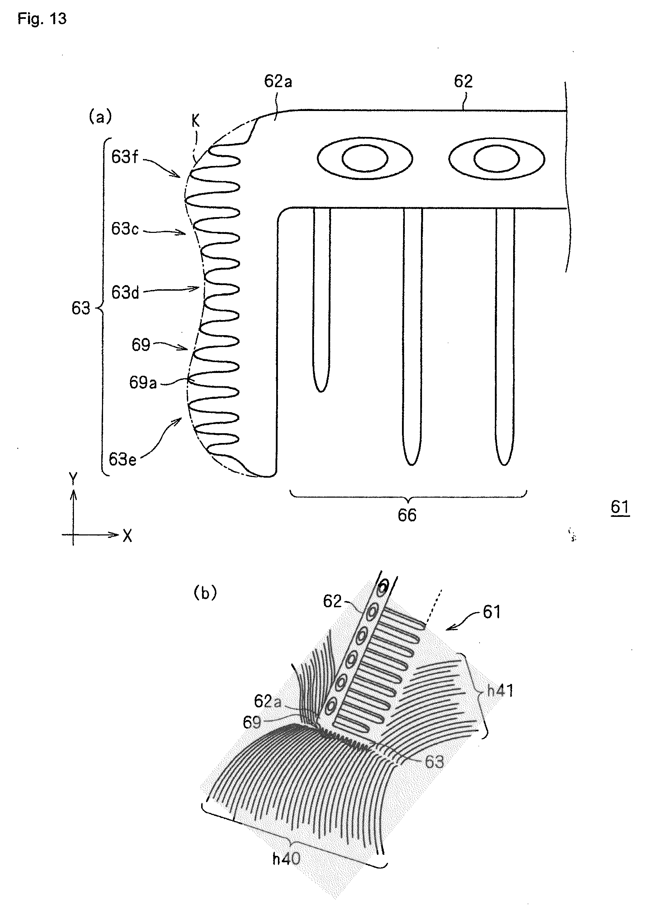

Figs. 13(a) and (b) illustrate a comb 61 according to a 7th embodiment of the present invention. Although the comb 61 of the 7th embodiment is equivalent to the comb 1 of the 1 st embodiment in its fundamental parts, it is characterized in that a comb part 69 having a plurality of comb teeth 69a is formed in one end edge comb part 63. In this comb part 69 one end outer edge surface 63c is formed with a locus K (illustrated by a dashed dotted line K in Fig. 13(a)) indicative of the positions of tip ends of respective comb teeth 69a. In other words, the one end outer edge surface 63c is formed with a concave portion by shortening the comb teeth 69a at part corresponding to one end dented portion 63d so as to have a shape equivalent to the one end outer edge surface 3c of the comb 1 of the 1 st embodiment. Further, the comb teeth 69a at part corresponding to outer edge portions 63e and 63f are lengthened to protrude respective tip ends so that convexed outer edge portions 63e and 63f are formed.

By configuring the comb part 69 as described above, even if the tip ends of the respective comb teeth 69a are pressed against the scalp Ha as illustrated in Figs. 4, 5, etc., the measurements of the length of hair (the hair bundle) can be carried out, while securing the state of two-point contact, similarly to the comb 1 illustrated in Fig. 1 etc. Note that since the respective comb teeth 69a are pressed against the scalp Ha, if they are considered to be used for the head part of the human body, it is preferred that the shape of the tip ends is rounded to some extent, rather than a pointed shape. Further, although the teeth lengths of the respective comb teeth 61a are about 5 mm for the shortest and about 8 mm for the longest in the example illustrated in Fig. 13(a), they are not limited to these teeth lengths but the value range from about 1 mm to about 10 mm can be applied, and it is preferred to secure the teeth lengths of 3 mm or longer if the comb function (the function to push the hair aside) is particularly desired to be certainly demonstrated. Note that a comb main body 62 is naturally provided with normal comb teeth 66.

Fig. 13(b) is a schematic perspective view illustrating a state of use of the comb 61. Since the comb 61 has the comb part 69 in the one end edge comb part 63, when the measurements of the length of hair are carried out, hair bundles h40 and h41 can be pushed aside by the respective comb teeth 69a of the comb part 69 at the time of pressing the one end outer edge surface 63c against the scalp Ha. Further, since respective hairs which constitute the pushed-aside hair bundles h40 and h41 get caught between the respective comb teeth 69a, the tip ends of the respective comb teeth 69a can contact with the scalp Ha. Thus, even in the case where the hair (hair bundle) of the head part with many amounts of hair is measured, the comb 61 allows correct measurements of the hair length by certainly pressing the one end outer edge surface 63c (the tip ends of the respective comb teeth 69a corresponding to the outer edge portions 63e and 63f) against the scalp after the hair is pushed aside by the comb part 69.

Note that, in the comb 61 according to the 7th embodiment, as for the indexes according to the measurements, corresponding portions in the respective embodiments described above can suitably be used. Further, as long as the respective comb teeth 69a of the comb part 69 are given flexibility, they can conform to the shape of the scalp to some extent. Therefore, the tip ends of the respective comb teeth 69a may be formed so that they are aligned linearly. Further, the parting work as illustrated in Fig. 3 can suitably be carried out by the comb 61 according to the 7th embodiment with the dented portion 63d.

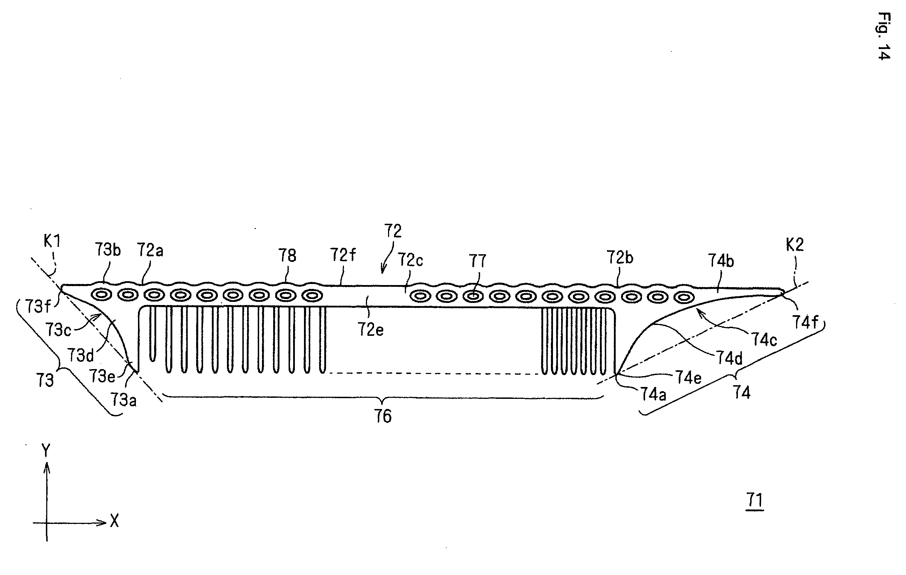

Fig. 14 illustrates a comb 71 according to an 8th embodiment of the present invention. The comb 71 of the 8th embodiment is characterized by being formed in a shape so that one end outer edge surface 73c which is the outer circumference side of one end edge comb part 73 provided on one end side 72a of comb main body 72 inclines about 45° with respect to the X-axis direction, and in a shape so that the other end outer edge surface 74c which is the outer circumference side of the other edge comb part 74 provided on the other end side 72b inclines about 22.5° with respect to the X-axis direction.

In other words, the one end edge comb part 73 is formed obliquely so that the one end outer edge surface 73c is along an inclination line K1 which is inclined by 45° with respect to the X-axis direction, and, thereby, the entire shape of the one end edge comb part 73 is formed in a triangular shape. In addition, the one end edge comb part 73 is formed with one end dented portion 73d between an outer edge portion 73e on a tip end 73a side and an outer edge portion 73f on a base end 73b side.

Further, the other end edge comb part 74 is configured so that the other end outer edge surface 74c is obliquely formed along an inclination line K2 which is inclined by 22.5° with respect to the X-axis direction, and, thereby, the entire shape of the other end edge comb part 74 also has a sharp triangular shape. Moreover, the other end edge comb part 74 is formed with the other end dented portion 74d between an outer edge portion 74e on a tip end 74a side and an outer edge portion 74f on a base end 74b side.

Note that, in the comb 71, a plurality of through-holes 77 which serve as indexes indicative of distances are formed in the comb main body 72 similarly the comb 1 of the 1 st embodiment. However, since the one end edge comb part 73 and the other end edge comb part 74 are a triangular shape, respectively, and since the one end edge comb part 73 and the other end edge comb part 74 have a large area compared with the case of the 1 st embodiment, the through-holes 77 are formed also in the one end edge comb part 73 and the other end edge comb part 74. Further, in the comb 71, convex portions 78 are formed in an upper surface 72f of the comb main body 72 so that the centers of the convex portion 78 corresponds to the centers of the through-hole 77, and each of the convex portions 78 is also an index indicative of a distance. These indexes indicate distances from the one end outer edge portion 73c, and also indicate distances from the other end outer edge portion 74c. Note that in the comb main body 2, the side surfaces and an upper surface 72f at a center part 72e in longitudinal directions are formed in flat to use them as a grip part.

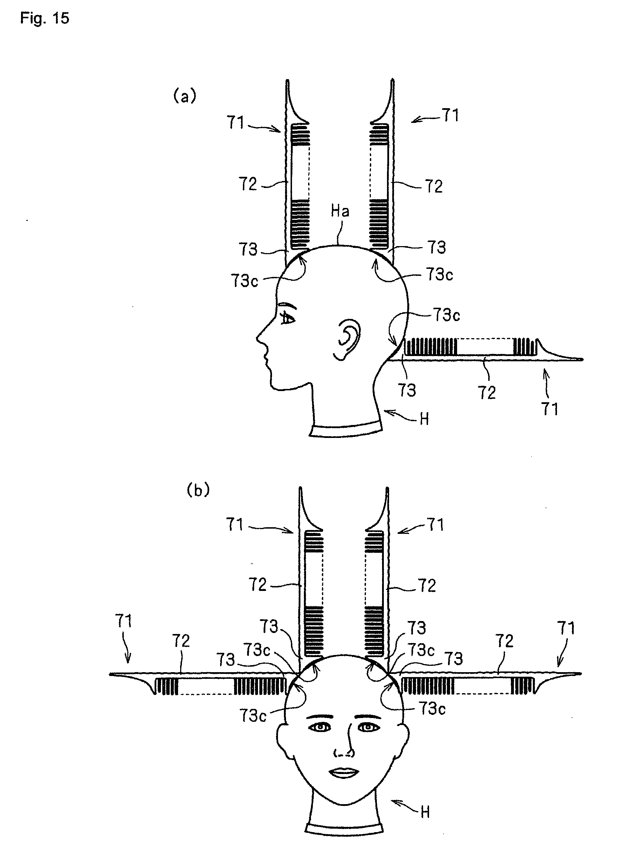

Figs. 15(a) and (b) illustrate a situation where the comb 71 of the 8th embodiment is used for the human head model H. Note that in Figs. 15(a) and (b), in order to clarify a feature of the state of use, illustration of the entire implanted hair is omitted.

Fig. 15(a) is a view seen from the side of the human head model H, and illustrates a case where the one end outer edge surface 73c of the one end edge comb part 73 is pressed against the scalp. For example, when measuring the length of a hair bundle implanted near the neck in a rear portion of the head part of the human head model H, the comb 71 is pressed against the scalp at the one end edge comb part 73 in a posture in which a comb teeth 76 are oriented upwardly. Here, the scalp Ha near the neck in the rear portion of the head part of the human head model H is a downwardly oblique curved surface which is curved downwardly toward the left as illustrated in Fig. 15(a). When the one end outer edge portion 73c which is inclined at 45° is pressed against the scalp Ha having such a downwardly curved surface, the comb 71 becomes in a substantially horizontal posture, and this posture can be stabilized by the one end dented portion 73d.

When measuring the length of a hair bundle implanted near a front part or near a rear part of the top portion of the human head model H, if the one end outer edge surface 73c which is inclined at 45°, of the one end edge comb part 73 is pressed against the scalp, the comb 71 becomes in a posture in substantially vertical directions, and this posture can be stabilized by the one end dented portion 73d also in such a case.

Note that since the posture of the comb 71 becomes in the substantially horizontal directions or in the substantially vertical directions at the time of measurement, the comb 71 becomes easy to use as a ruler which matches with users' (a beautician or a barber) sense of direction. In other words, when the user (a beautician or a barber) carries out the work, such as cutting the hair or setting the hair style, he/she uses unconsciously, as the ruler in the substantially vertical directions or the substantially horizontal directions, a line indicating the vertical directions or a line indicating the horizontal directions which exists in the circumference of the work place, such as a vertical line or a lateral line of the frame of a rectangular mirror, or a vertical line or a horizontal line of a cabinet. Therefore, since in the comb 71, the posture in the state where the one end outer edge portion 73c is pressed against the head part can be oriented in the substantially horizontal directions or in the substantially vertical directions, the directions match with the vertical line in the vertical directions or the lateral line in the horizontal directions, which are used as the ruler existing in circumference of the user as described above. Thus, the directions match with the user's sense of direction, and the comb 71 is effective for user-friendliness in practical (refer to Figs. 15(a) and (b)).

Fig. 15(b) is a view seen from the front of the human head model H, and illustrates a state where the one end outer edge surface 73c of the comb 71 is pressed against the scalp. Also in such a case, since the posture of the comb 71 at the time of measuring the length of the hair bundle implanted near the left-and-right temporal regions, and the right side and the left side of the top portion of the human head model becomes in substantially horizontal directions or in substantially vertical directions, the directions match with the user's sense, and the posture can be stabilized by the one end dented portion 73d (note that illustration of the hair is omitted in Fig. 15).

Further, practical skill assignments in which the head hair is pulled out at about 45° from the scalp and is cut by a predetermined length may generally be given, for example, for practical skill instructions at a barber school or a beauty school, or practical skill official examinations of hairdressing and beauty. In such a case, the method of measuring with the comb 71 as illustrated in Figs. 15(a) and (b) matches with such practical skill problems.

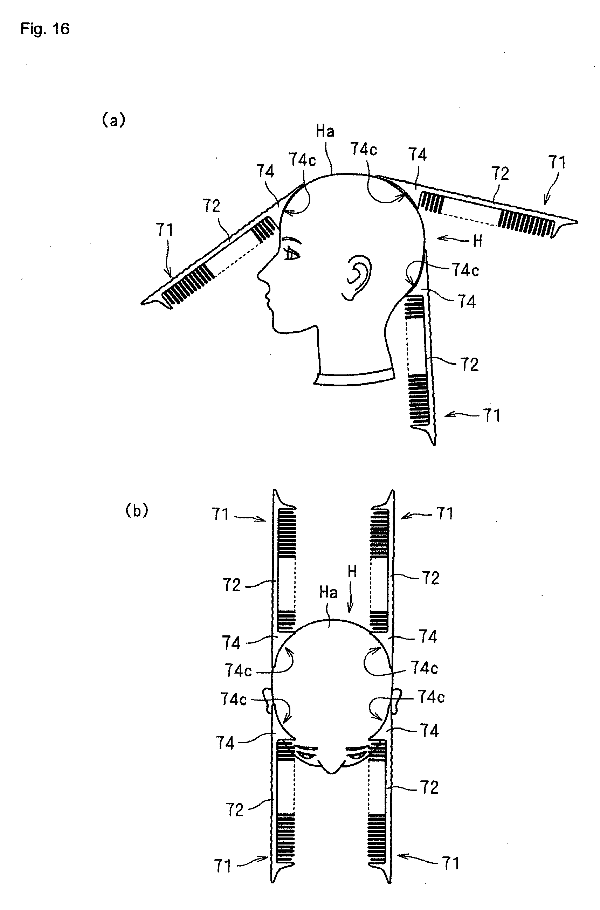

Further, Fig. 16(a) is a view seen from the side of the human head model H, and Fig. 16(b) is a view seen from above the human head model H. Both the figures illustrate a case where the other end outer edge surface 74c of the other end edge comb part 74 is pressed against the scalp Ha. Also in such a case, measurements of the length of the head hair can be carried out in a direction which is inclined at 22.5° with respect to the scalp Ha by the other end outer edge surface 74c which is inclined at 22.5°, and the posture at the time of measurement can be stabilized by the other end dented portion 74d. Further, practical skill assignments in which the hair is pulled out at about 22.5° from the scalp and is cut may be given, for example, for practical skill instructions at a barber school or a beauty school, or practical skill official examinations of hairdressing and beauty. In such a case, the method of measuring by the comb 71 as illustrated in Figs. 16(a) and (b) matches with such practical skill problems etc. As described above, the comb 71 of the 8th embodiment is advantageous in that the measurements of length according to the two angles of inclination can be carried out by the single comb 71.

Note that, the comb 71 according to the 8th embodiment is not limited to the form as described above, but other values may of course be applied to the inclination angles of the one end outer edge surface 73c and the other end outer edge surface 74c. Further, if the specification of the comb 71 is to be simplified, it is also possible to form either the one end outer edge portion 73c or the other end outer edge portion 74c in the configuration same as the conventional art. In such a case, the measurements of length of the head hair can be carried out at the inclined angle by one of the inclined outer edge surfaces. Also in the comb 71 of the 8th embodiment, it is possible to suitably use the indexes of the respective embodiments described above. Further, the comb 71 according to the 8th embodiment can also suitably allow the parting work as illustrated in Fig. 3 by the one end dented portion 73d of the one end edge comb part 73.

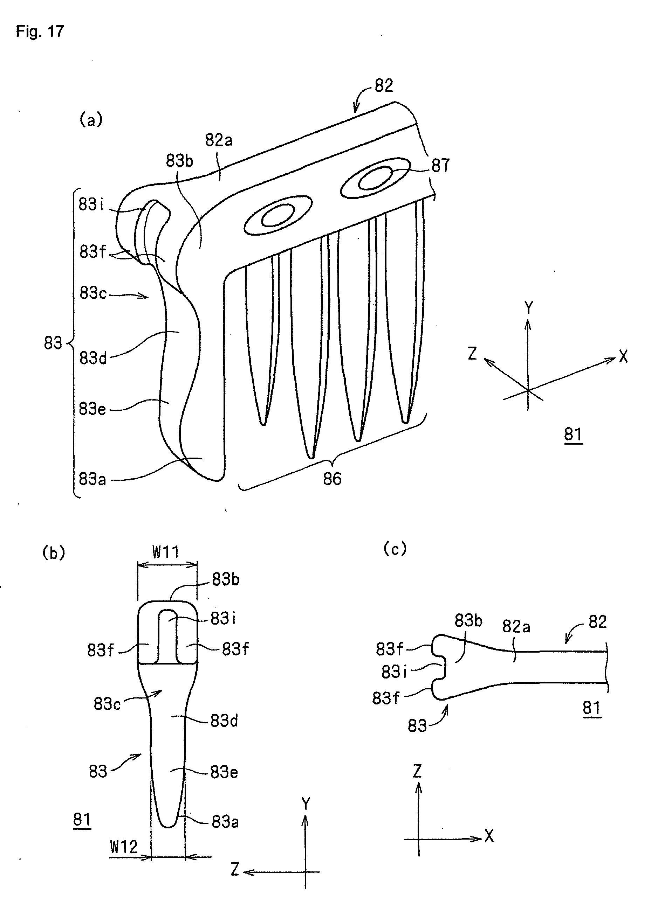

Fig. 17(a) illustrates a comb 81 according to a 9th embodiment of the present invention. The comb 81 of the 9th embodiment is characterized in that a concave portion 83i is formed in an outer edge portion 83f on a base end 83b side of one end edge comb part 83 provided on one end side 82a of a comb main body 82 from which a plurality of comb teeth 86 are extended. Note that the fundamental shape itself of the one end edge comb part 83 is equivalent to the comb 1 of the 1 st embodiment. In the one end edge comb part 83, one end dented portion 83d is formed in a center part of the one end outer edge surface 83c in the Y-axis direction, and convexed outer edge portions 83e and 83f are formed in a tip end 83a and a base end 83b, respectively.

As illustrated in Fig. 17(b), the one end edge comb part 83 is formed so that the thickness thereof in the Z-axis directions is larger at the base end 83b side than the tip end 83a side, where the thickness of the tip end 83a is W12 and the thickness of the base end 83b is W11 (W11>W12).

Further, as illustrated in Figs. 17(a), (b), and (c), the concave portion 83i is formed in the center part in the thickness directions (Z-axis direction) of the outer edge portion 83f of the one end outer edge surface 83c on the base end 83b side. The outer edge portion 83f of the base end 83b has a shape which is divided into two parts by the concave portion 83i.

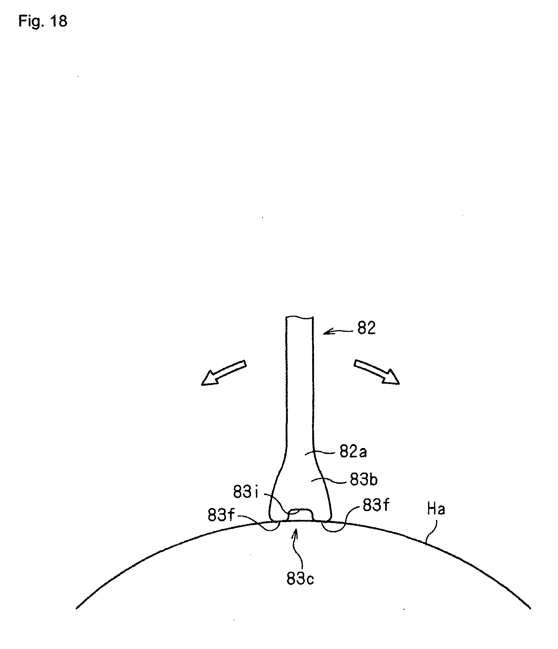

Fig. 18 illustrates a state where the one end outer edge portion 83c of the one end edge comb part 83 is pressed against the scalp Ha. In such a case, as described above, since the outer edge portion 83f of the base end 83b is divided into the two parts by the concave portion 83i, a total of three locations including the outer edge portion 83e of the tip end 83a contact the scalp Ha. As a result, the comb 81 can secure the state of the three-point contact, the shaking in the directions (the Y-axis directions) described in Fig. 6 of the comb 1 of the 1st embodiment can be prevented, the shaking in the thickness directions (the Z-axis directions) illustrated by the white arrows in Fig. 18 can also be prevented, and a more stable posture can be maintained. Thereby, the measurements of the length of the hair can be carried out more correctly by a plurality of through-holes 87 formed in the comb main body 82.

Note that in the comb 81 according to the 9th embodiment, the indexes of the respective embodiments described above can be used suitably as the indexes for the measurements. Further, it is possible to use the configurations of the 1 st embodiment to the 3rd embodiment as described above are suitably as the configuration on the other end side of the comb main body 82. Further, the configurations of the respective parts of the comb 1 according to the 1st embodiment to the comb 81 according to the 9th embodiment as described above can also be combined suitably. Further, the comb 81 according to the 9th embodiment also allows the suitable parting work as illustrated in Fig. 3 by the one end dented portion 83d of the one end edge comb part 83.

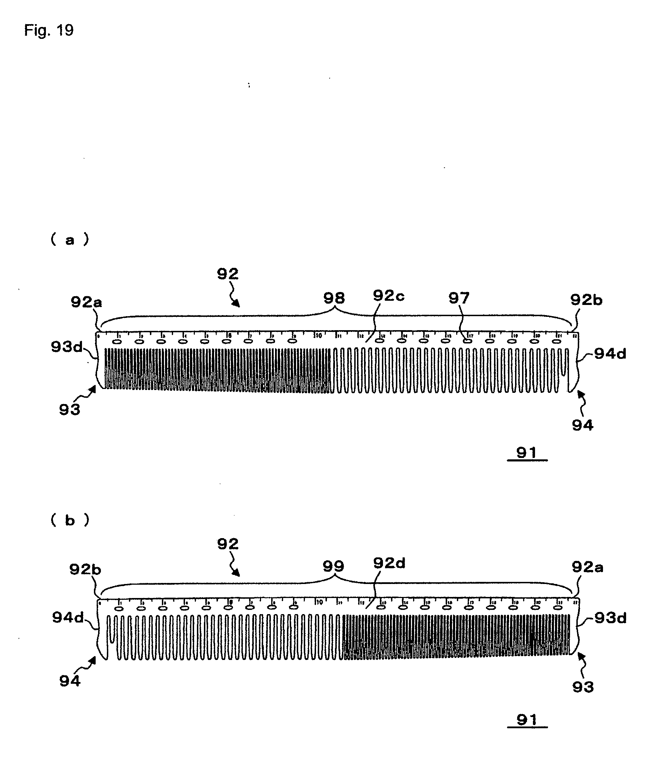

Figs. 19(a) and (b) illustrate a comb 91 according to a 10th embodiment. The comb 91 is characterized in that scales 98 and 99 are respectively given to one side surface 92c and the other side surface 92d of a comb main body 92 where a plurality of through-holes 97 are formed. The scales 98 and 99 are comprised of long lines with a 10-mm interval and short lines indicative of a dimension of 5 mm between the respective long lines. In order to measure a dimension from both sides of one end side 92a and the other end side 92b of the comb main body 92, the respective long lines and short lines are drawn so that the dimensions from both the ends on the one end side 92a and the other end side 92b are indicated. Note that the centers of the through-holes 97 formed in the comb main body 92 are located corresponding to the long lines indicative of the 10-mm interval of the scales 98 and 99, and the respective through-holes 97 also function as indexes indicative of the 10-mm interval is illustrated. Further, in the comb 91, one end edge comb part 93 which is formed with one end dented portion 93d and the other end edge comb part 94 formed with the other end dented portion 94d are formed at one end side 92a and the other end side 92b of the comb main body 92, to allow stable measurements of the head hair by either side, while allowing the parting work as illustrated in Fig. 3 by either side (since the closest comb tooth to the other end edge comb part 94 is short, the parting work is especially easy).

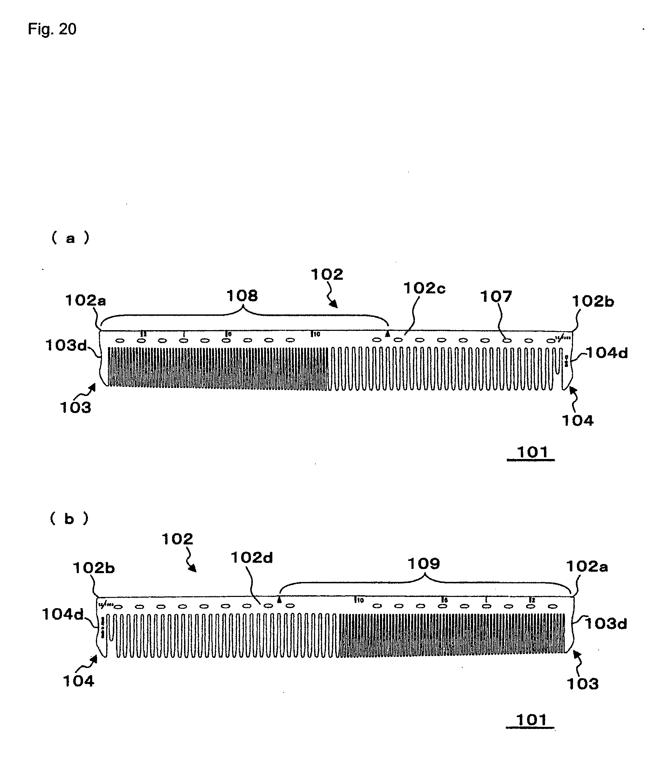

Figs. 20(a) and (b) illustrate a comb 101 according to an 11th embodiment, and the comb 101 differs from the comb 91 of the 10th embodiment in the forms of the scales. In other words, scales 108 and 109 are respectively given to one side surface 102c and the other side surface 102d of a comb main body 102, and both the scales 108 and 109 are configured to allow measurements of a dimension from one end 102a side of the comb main body 102. Further, the scales 108 and 109 are not for measurements of a fine dimension like the 10th embodiment (10 mm, 5 mm), but the scales 108 and 109 indicate only dimensions which are commonly and often used for practical skill official examinations, actual works in beauty parlors, etc. (2 cm, 4 cm, 6 cm, 10 cm, etc.), and, thereby, the scales are configured to indicate necessary dimensions to be easily recognizable at a glance. Note that in the comb 101, one end edge comb part 103 formed with one end dented portion 103d and the other end edge comb part 104 formed with the other end dented portion 104d are formed at the one end side 102a and the other end side 102b of the comb main body 102, to allow a suitable parting work as illustrated in Fig. 3 by either side (since the closest comb tooth to the other end edge comb part 94 is short, the parting work is especially easy).

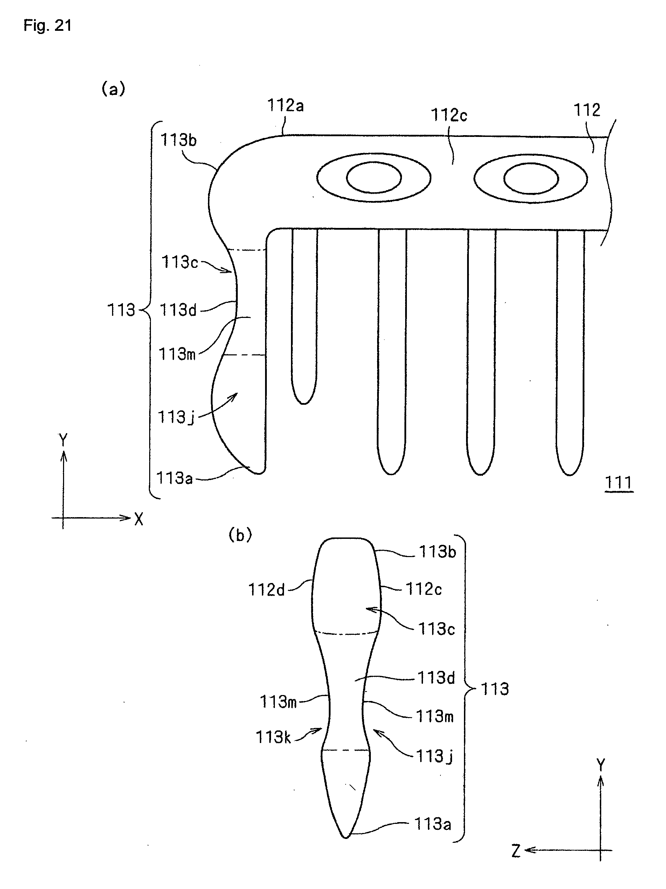

Figs. 21(a) and (b) illustrate a comb 111 according to a 12th embodiment. This comb 111 is characterized in that side dented portions 113m and 113n are formed in side surfaces 113j and 113k of one end edge comb part 113 formed on one end 112a side of a comb main body 112.

First, in the one end edge comb part 113, one end dented portion 113d is formed in one end outer edge surface 113c, similarly the comb 1 of the 1st embodiment etc. as described above. Further, in the one end edge comb part 113, the one side dented portion 113m is formed in the side surface 113j which faces the same direction as one side surface 112c of the comb main body 112, in a center part between a tip end 113a side and a base end 113b side (the one side dented portion 113m is dented parallel to the Z-axis direction (thickness direction)). Further, in the one end edge comb part 113, the other side dented portion 113n is formed in the side surface 113k which faces the same direction as the other side surface 112d of the comb main body 112, in a center part between the tip end 113a side and the base end 113b side (the other side dented portion 113n is dented parallel to the Z-axis direction (the thickness direction)).

The one end dented portion 113d of the one end outer edge surface 113c, the side dented portion 113m of the one side surface 113j, and the side dented portion 113n of the other side surface 113k are formed at the same location in the Y-axis directions, and, thereby, the respective dented portions 113d, 113m, and 113n have a form so that the respective dented portions 113d, 113m, and 113n communicate with each other. Therefore, the one end edge comb part 113 has a shape which is narrowed at three sides of the one end outer edge surface 113c, the one side surface 113j, and the other side surface 113k, in the center part between the tip end 113a side and the base end 113b side. Note that the comb 111 according to the 12th embodiment has a form equivalent to the comb 1 of the 1st embodiment etc. for other than the parts described above.

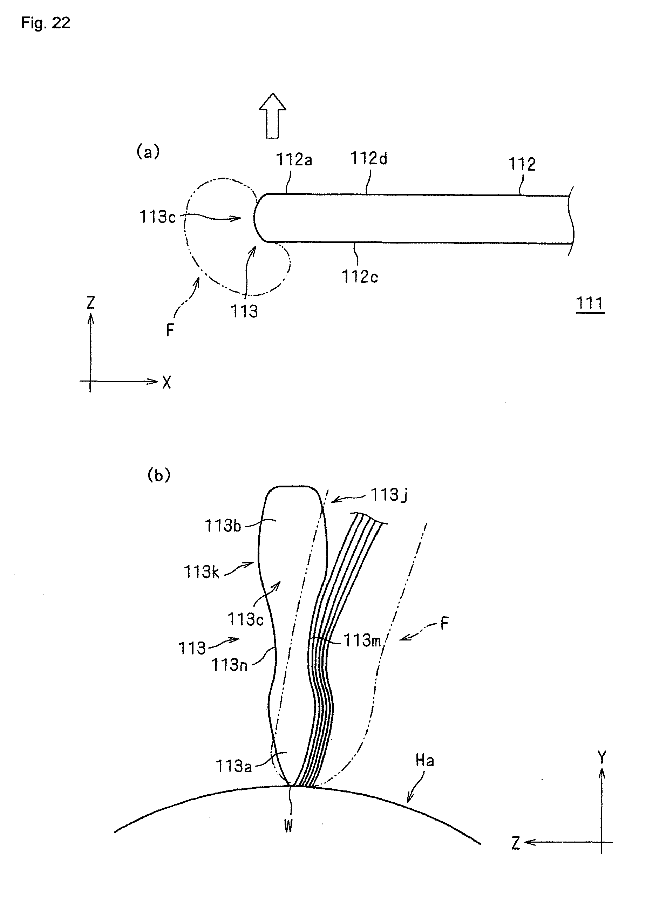

Fig. 22(a) and (b) illustrate one example of a case of carrying out a parting work using the one end edge comb part 113 of the comb 111. When carrying out the parting work, there are, for example, some users who apply his/her finger, which is applied to the one end edge comb part 113, to two sides from the one end outer edge surface 113c to one side surface 113j, in addition to applying his/her finger to the one end outer edge surface 113c, and some users who apply his/her finger to two sides from the one end outer edge surface 113c to the other side surface 113k, some users who apply to three sides of the one end outer edge surface 113c, the one side surface 113j, and the other side surface 113k. Such various ways of applying the finger are caused by reasons, such as the user is right-handed or left-handed, the thickness of the user's finger, the user's taste of the method of holding the comb, etc.

Figs. 22(a) and (b) illustrate, among the various finger applying methods described above, an example of applying the finger to two sides from the one end outer edge surface 113c to the one side surface 113m, where Fig. 22(a) is a view seen from above the comb, and Fig. 22(b) is a view seen from the one end outer edge surface 113c. When carrying out a parting work, a finger F applied to the one end edge comb part 113 which is pressed at a tip end 113a thereof against the parting line W to be formed in the scalp Ha covers two sides from the one end outer edge surface 113c to the one side surface 113j and pinches a hair bundle divided outwardly by the one end edge comb part 113 between the two circumferential sides of the one end edge comb part 113 and the finger F. The hair bundle pinched between the two sides of the one end edge comb part 113 and the finger F is spreads naturally within a space formed between the one end dented portion 113d and the one side dented portion 113m.

Further, since the respective parts on the tip end 113a side and the base end 113b side which are located on both sides of the one end dented portion 113d and the one side dented portion 113m (parts located on both sides in the Y-axis directions) are convexed in the two sides of the one end edge comb part 113, the user's finger F contacts centering on these convexed parts. Thus, a gap is produced between the finger F and the outer circumferential surface of the one end edge comb part 113. Therefore, it can also be reduced that the hair bundle pinched between the finger F and the outer circumferential surface of the one end edge comb part 113 tends to be stuck, because of the existence of the gap.

As described above, the tendency of the hair bundle between the one end edge comb part 113 and the finger F being stuck over a large range in the Y-axis directions like the conventional arts are eliminated. Further, when the one end edge comb part 113 is moved toward an arrow illustrated in Fig. 22(a) along the line where the parting line W is to be formed, the resistance due to the sticking of the hair bundle is significantly reduced compared with the conventional arts, and, thereby, the one end edge comb part 113 can smoothly be moved. Accordingly, the situation where some hairs are fallen out from the hair bundle divided outside the one end edge comb part 113 will not be caused, and, thereby, the clear parting line W can be formed.

The details based on Figs. 22(a) and (b) are examples, and when the finger is applied to the two sides from the one end outer edge surface 113c to the other side surface 113k, and when the finger is applied to the three sides of the one end outer edge surface 113c, the one side surface 113j, and the other side surface 113k, the resistance due to the sticking of the hair bundle is reduced, the one end edge comb part 113 is moved smoothly, and the efficient parting work can be carried out, as similarly the above details of Figs. 22(a) and (b). Note that the comb 111 of the 12th embodiment secures functions equivalent to the comb 1 of the 1st embodiment etc. for other than the details described above, and, of course, it is also possible to press the one end outer edge surface 113c of the one end edge comb part 113 against the scalp to correctly measure the length of the hair.

Further, the comb 111 according to the 12th embodiment may be configured such that the side dented portions 113m and 113n are formed only in either one of the side surfaces 113j and 113k on both sides of the one end edge comb part 113. If the side dented portion is formed in only either one of the side surfaces 113j and 113k, it becomes suitable for the user who carries out the parting work by applying his/her finger to the side surface 113 on the side where the side dented portion is formed. In addition, since the dimension in the thickness directions (the Z-axis directions) of the one end edge comb part 113 becomes thicker than the case where the side dented portions 113m and 113m are formed on both the side surfaces 113j and 113k, it is easier to secure the rigidity of the one end edge comb part 113.

The configurations, the modifications, etc. of the 1 st to 11 th embodiments described above can also be applied to the comb 111 according to the 12th embodiment, and, for example, the various forms described above are applicable to the indexes required for the measurements of length. Further, the comb 111 of the 12th embodiment may be configured so that the other end edge comb part on the other end side of the comb main body 112 is formed equivalent to the one end edge comb part 113 which is formed with the side dented portions described above, and, by configuring in this way, the parting work can favorably be carried out even if either one of the comb edge parts on both sides are used.

### INDUSTRIAL APPLICABILITY

The present invention is suitably available for allowing the comb to be used not only for hairdressing but for the parting work in which the beautiful parting line is efficiently produced, and for assistance of a precise hair cut by allowing the correct measurements of the length of hair (hair bundle).

### DESCRIPTION OF REFERENCE NUMERALS

- 1:: Comb
- 2:: Comb Main Body
- 3:: One End Edge Comb Part
- 3c:: One End Outer Edge Surface
- 3d:: One End Dented Portion
- 3e, 3f:: Outer Edge Portion
- 4:: The Other End Edge Comb Part
- 4c:: The Other End Outer Edge Surface
- 4d:: The Other End Dented Portion
- 4e, 4f:: Outer Edge Portion
- 7:: Through-Hole
- 15:: Grip Part
- 38:: Concave-Convex Part
- 38a:: Mountain Portion
- 38b:: Valley Portion
- 58, 59:: Curved Part
- 69:: Comb Part
- 83i:: Concave Portion
- 113m, 113n:: Side Dented Portion

## Claims

1. A comb comprising a comb main body, one end edge comb part formed on one end side in a longitudinal direction of the comb main body, and a plurality of comb teeth formed on the comb main body, wherein
a dented portion is formed in an outer edge surface between a tip end side and a base end side of the one end edge comb part.

2. The comb as recited in claim 1, wherein
the comb main body has indexes indicative of distances parallel to the longitudinal directions as the outer edge surface of the one end edge comb part is pressed against a scalp to contact the outer edge portions that remain on both sides of the dented portion with the scalp, wherein
a measurement of a length of head hair is carried out based on the indexes by applying the outer edge surface of the one end to the scalp to contact.

3. The comb as recited in claim 2, wherein
a grip part is formed on the other end side of the comb main body, wherein
an outer edge dented portion is formed in an outer edge surface of the grip part, and wherein
the measurements of the length of the head hair is carried out based on the indexes by applying the outer edge surface of the grip part to the scalp to contact the outer edge portions that remain on both sides of the outer edge dented portion with the scalp.

4. The comb as recited in claim 2, comprising
the other end edge comb part formed on the other end side in the longitudinal directions of the comb main body, wherein
the other end dented portion is formed in an outer edge surface between a tip end side and a base end side of the other end edge comb part, and wherein
the measurements of the length of the head hair is allowed to be carried out based on the indexes by applying the outer edge surface of the other end edge comb part to the scalp to contact the outer edge portions that remain on both sides of the other end dented portion with the scalp.

5. The comb as recited in claims 2 to 4, wherein
the outer edge surface of the one end edge comb part is formed obliquely with respect to the longitudinal directions of the comb main body.

6. The comb as recited in claim 4, wherein
the outer edge surface of the one end edge comb part and the outer edge surface of the other end edge comb part are formed obliquely with respect to the longitudinal directions of the comb main body, respectively, and wherein
an angle of the oblique outer edge surface of the one end edge comb part is different from an angle of the oblique outer edge surface of the other end edge comb part.

7. The comb as recited in claim 4 or 6, wherein
the comb main body has first indexes indicative of distances from the one end side of the comb main body and second indexes indicative of distances from the other end side of the comb main body.

8. The comb as recited in any one of claims 2 to 7, wherein
the comb main body has first indexes corresponding to a first distance unit system and second indexes corresponding to a second distance unit system.

9. The comb as recited in claim 7 or 8, wherein
the first indexes are different in appearance from the second indexes.

10. The comb as recited in any one of claims 1 to 9, wherein
a comb part having a plurality of comb teeth is provided to the one end edge comb part, and tip ends of the respective comb teeth of the comb part form the outer surface.

11. The comb as recited in any one of claims 1 to 10, wherein
a thickness of the one end edge comb part is larger at the base end side than the tip end side, and wherein
a concave portion is formed in the outer edge surface at a center portion in thickness directions, on the base end side of the one end edge comb part.

12. The comb as recited in any one of claims 1 to 11, a side dented portion is formed in the one end edge comb part, in at least one of side surfaces between the tip end side and the base end side.
